Europäisches Patentamt

European Patent Office

Office européen des brevets

(19)

(11) Numéro de publication : **0 444 183 B1**

(12) **FASCICULE DE BREVET EUROPEEN**

(45) Date de publication du fascicule du brevet :
25.10.95 Bulletin 95/43

(51) Int. Cl.⁶ : **G06T 17/20**

(21) Numéro de dépôt : **90914214.3**

(22) Date de dépôt : **19.09.90**

(86) Numéro de dépôt international :
**PCT/FR90/00676**

(87) Numéro de publication internationale :
**WO 91/04544 04.04.91 Gazette 91/08**

(54) PROCEDE ET DISPOSITIF DE MODELISATION D'UNE SURFACE.

(30) Priorité : **20.09.89 FR 8912341**

(43) Date de publication de la demande :
**04.09.91 Bulletin 91/36**

(45) Mention de la délivrance du brevet :
**25.10.95 Bulletin 95/43**

(84) Etats contractants désignés :
**AT BE CH DE DK ES FR GB IT LI LU NL SE**

(56) Documents cités :
**EP-A- 0 196 651**
**Comput. & Graphics, Volume 6, No. 2, 1982,**
**Pergamon Press Ltd, (Oxford, GB), S.SINGH et**
**al.: "FEM Shape Optimization: A Case for**
**Interactive Graphics", pages 63-72 voir page**
**65, colonne de gauche, ligne 34 - colonne de**
**droite, ligne 8**

(73) Titulaire : **ASSOCIATION SCIENTIFIQUE**
**POUR LA GEOLOGIE ET SES APPLICATIONS**
**94, avenue De Lattre-de-Tassigny**
**F-54001 Nancy Cedex (FR)**
Titulaire : **SOCIETE NATIONALE ELF**
**AQUITAINE (PRODUCTION)**
**Tour Elf,**
**2, Place de la Coupole,**
**La Défense 6**
**F-92400 Courbevoie (FR)**
Titulaire : **TOTAL COMPAGNIE FRANCAISE**
**DES PETROLES**
**24, Cours Michelet**
**F-92800 Puteaux (FR)**
Titulaire : **COMPAGNIE GENERALE DE**
**GEOPHYSIQUE**
**1, Rue Léon Migaux**
**F-91341 Massy Cédex (FR)**

(72) Inventeur : **MALLET, Jean-Laurent**
**8, rue de la Croix-Gagnée**
**F-54000 Nancy (FR)**

(74) Mandataire : **Martin, Jean-Jacques et al**
**Cabinet REGIMBEAU**
**26, Avenue Kléber**
**F-75116 Paris (FR)**

EP 0 444 183 B1

## Description

La présente invention concerne d'une façon générale l'investigation dans des corps à trois dimensions, et concerne en particulier un nouveau procédé pour l'obtention d'une représentation d'une surface située au sein de ce corps à partir d'un ensemble limité de données géométriques connues relatives à ladite surface.

L'investigation dans des corps tridimensionnels est une préoccupation majeure tout particulièrement dans les domaines de la géologie, de la géophysique et de la biologie.

Ainsi, par exemple dans le domaine de la géophysique, on souhaite obtenir des représentations aussi fidèles que possibles de surfaces situées par exemple à l'interface entre deux zones de natures ou de propriétés différentes, à partir de données obtenues lors de campagnes de prospection, voire en cours d'exploitation d'une richesse souterraine.

En effet, l'efficacité de l'investigation du corps tridimensionnel, et en particulier de la recherche de pétrole ou autre richesse souterraine, est conditionnée par la fidélité avec laquelle on peut reconstituer et représenter ce genre de surface.

Par ailleurs, dans le domaine médical et biologique, l'on sait actuellement obtenir, par des procédés divers, des représentations de coupes d'un corps vivant ou analogue, et l'on recherche également à reconstituer et à représenter, de façon tridimensionnelle, une surface située à l'interface entre deux milieux, et notamment les contours d'un organe ou autre.

Il existe à l'heure actuelle un certain nombre de techniques de modélisation permettant d'obtenir la représentation d'une surface dans un corps à trois dimensions que l'on cherche à exploiter ou à traiter. On citera en particulier les interpolations de Bézier ou les fonctions splines (on se référera pour plus de détails par exemple à l'ouvrage Geometric Modelling, M. E. Mortenson, Ed. John Wiley, 1985).

On connaît également par le document "Geometrical modelling and Geostatistics", Proceedings of the Third International Geostatistics Congress, Kluwer Academic Publisher, 1989, un procédé conforme au préambule de la revendication 1.

Cependant, ces techniques connues sont toutes très mal adaptées à la prise en compte de données hétérogènes, à savoir des données relatives à la fois aux coordonnées de points de la surface, à des orientations imposées de la surface, à des liens géométriques entre deux points distants, etc. En outre, ces techniques sont inadaptées à une trop grande dispersion des surfaces ou encore à des anomalies de surfaces telles que des plis, des cassures, ou encore des surfaces discontinues. Plus précisément, dans certains cas, les fonctions mises en oeuvre dans ces techniques soit ne convergent pas, soit n'admettent aucune solution, soit admettent au contraire une pluralité de solutions.

Enfin les techniques connues n'offrent pas la possibilité de prendre en compte la notion de plus ou moins grande certitude avec laquelle certaines données de la surface sont connues.

La présente invention vise à pallier les inconvénients des procédés de la technique antérieure et à proposer un procédé qui puisse prendre en compte des informations géométriques non homogènes et/ou très dispersées, ainsi que les anomalies précitées et d'autres en offrant à chaque fois une modélisation unique et sans équivoque de la surface.

Un autre objet de l'invention est de proposer un procédé qui puisse s'appuyer sur des données géométriques en prenant en compte simultanément un degré de certitude ou de précision lié à ces données.

A cet effet, la présente invention concerne tout d'abord un procédé d'obtention d'une modélisation d'une surface telle que définie dans la revendication 1.

Des aspects préférés de ce procédé sont définis dans les sous-revendications 2 à 16.

L'invention concerne également un dispositif pour l'obtention d'une modélisation d'une surface tel que défini dans la revendication 17.

D'autres aspects, buts et avantages de la présente invention apparaitront mieux à la lecture de la description détaillée suivante d'une forme de réalisation préférée de celle-ci, donnée à titre d'exemple non limitatif et faite en référence aux dessins annexés, sur lesquels :

la figure 1 est un exemple de graphe incorporant un maillage utilisé pour modéliser une surface, ce graphe étant défini par l'ensemble de tous les côtés de facettes dont les sommets constituent l'ensemble $\Omega$ décrit plus loin, et illustre également des flèches symbolisant des déplacements de noeuds du maillage effectués interactivement par l'utilisateur pour façonner la surface;

les figures 2(a) à 2(d) illustrent quatre cas de contrôle de la forme d'une surface à l'aide de quatre types différents de contraintes vectorielles; les vecteurs $\overrightarrow{\Delta\lambda_\mu}$ sont supposés connus directement sur les figures 2(a) à 2(c) et connus par leur orthogonalité à un vecteur $\overrightarrow{v}$ donné sur la figure 2(d);

la figure 3 illustre un exemple de surface géologique complexe (dôme de sel) modélisée à l'aide du procédé

2

de la présente invention;

la figure 4 illustre un exemple de surface biologique complexe (cerveau d'embryon) modélisée à l'aide du procédé de la présente invention à partir de données ponctuelles obtenues par une succession de coupes:

la figure 5 illustre l'organisation de la mémoire associée à un atome A(k) de noyau 7 $\overrightarrow{\varphi k}$ ;

la figure 6 illustre un premier exemple de mémorisation d'une orbite d'un atome donné, dans un tableau de $n_k$ cases;

la figure 7 illustre un second exemple de mémorisation d'une orbite d'un atome donné, dans $n_k$ tableaux chaînés de deux cases chacun;

la figure 8 illustre la mémorisation d'une donnée géométrique ou contrainte;

la figure 9 illustre la manière de mémoriser un ensemble de données géométriques ou contraintes relatives à un atome A(k) donné;

la figure 10 illustre la mémorisation dans un tableau de l'ensemble des atomes relatifs à une surface S; et

la figure 11 illustre l'enchainement de l'accès audit ensemble d'atomes.

On indiquera tout d'abord que, d'une figure à l'autre, des éléments ou parties identiques ou similaires sont désignés par les mêmes numéros de référence.

En outre, on va indiquer ci-dessous un certain nombre de publications présentant l'état de la technique relatif à la présente invention.

(1) A method of Bivariate interPolation and smooth surface fitting based on local procedures, H. AKIMA, J. ACM 17.1, 1974;

(2) Shape reconstruction from planar cross-sections, J.D. BOISSONNAT, ACM Transactions on Graphics, vol. No. 2, 1986

(3) Machine contouring using minimum curvature, I.C. BRIGGS, Geophysics 17,1, 1974;

(4) Triangular Bernstein-Bézier patches, G. FARIN, Computer-aided geometric design, 1986;

(5) Primitives for the manipulation of general subdivisions and the computation of Voronoi diagrams, L. GUIBAS et J. STOLFI, ACM Transactions on Graphis, vol. No. 2, 1985.

(6) Three-dimensional graphic display of disconnected bodies, J.L. MALLET, Mathematical Geology, vol. 20, no. 8, 1988;

(7) Geometrical modelling and geostatistics, J.L. MALLET, Proceedings of the Third International Geostatistics Congress, Kluwer Academic Publishers, 1989;

(8) Discrete Smooth Interpolation, J.L. MALLET, ACM Transactions on Graphics, April issue, 1989;

(9) Geometric Modelling, M.E. MORTENSON, John Wiley, 1989.

Par ailleurs, la description qui suit d'une forme de réalisation préférée de l'invention est elle-même suivie d'une annexe exposant une méthode de reconstitution d'une surface dérivée de la méthode exposée dans le document (8) et sur laquelle est basé le procédé de la présente invention.

## 1. DESCRIPTION DU PROCEDE DE CODAGE ET D'AJUSTEMENT DE SURFACES COMPLEXES SELON L'INVENTION

### 1.1. Introduction

1.1.1 Objet de la description

On va décrire ci-dessous un procédé industriel de codage et d'ajustement de surfaces complexes rencontrées par exemple lors de la modélisation d'interfaces entre zones de natures ou de propriétés différentes de couches géologiques ou de corps d'organismes biologiques.

Le résultat de ce codage peut être transcrit sous forme d'images et/ou de maquettes en matériel solide (par exemple plastique) à l'aide d'un ordinateur graphique ou de tout autre procédé. En géologie, par exemple, ces images et/ou maquettes sont ensuite utilisées pour optimiser l'exploration, l'exploitation et la gestion des ressources du sous-sol telles que :

. pétrole

. minerai

. eau

. etc.

Le procédé décrit peut être décomposé en deux sous-procédés complémentaires :

1. un sous-procédé de codage d'une surface

2. un sous-procédé d'ajustement d'une surface aux données disponibles.

En géologie, par exemple, ces données peuvent être :

- la position (exacte ou approchée) de points sur une coupe de la surface par un plan
- la position (exacte ou approchée) de l'intersection d'un forage avec la surface géologique correspondant à la séparation entre deux couches
- le plan tangent (exact ou approché) en un point donné de la surface correspondant à la séparation entre deux couches
- le rejet (exact ou approché) d'une faille (=cassure) affectant la surface correspondant à la séparation entre deux couches
- etc ...

Le sous-procédé de codage est conçu de façon à optimiser les performances du procédé d'ajustement basé sur une variante de la méthode DSI (cf ref. [8]) spécialement adaptée à la modélisation géométrique des surfaces. Cette variante de la méthode DSI utilisée ici n'a encore jamais été publiée et est présentée en annexe.

### 1.1.2 Décomposition d'une surface S en facettes

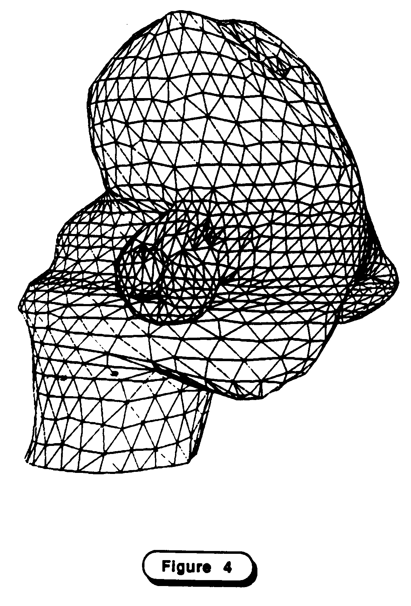

Soit $S$ une surface à modéliser. Comme le montre la Figure 4 et ainsi qu'il a été suggéré dans les références [6] et [7], celle-ci sera supposée approchée par un ensemble de facettes triangulaires et/ou polygonales. Ces facettes sont caractérisées par :

- La connaissance des coordonnées de leurs sommets constituant un ensemble fini de $N$ points $\{ \vec{\varphi}_1$ ,...,

$\vec{\varphi}_N \}$ régulièrement répartis sur $S$. Ces sommets sont numérotés de 1 jusqu'à $N$ et les coordonnées du $k$ème sommet $\vec{\varphi}_k$ seront notées $(\varphi_k^x, \varphi_k^y, \varphi_k^z)$

- La donnée de leurs côtés constitués par des liens entre couples de sommets ( $\vec{\varphi}_i$ , $\vec{\varphi}_j$ )

L'ensemble des côtés des triangles et/ou polygones composant une surface $S$ constitue un graphe $G$ dont les noeuds sont précisément les sommets des triangles et/ou polygones. Pour cette raison, et afin d'éviter des périphrases inutiles, nous utiliserons de façon équivalente le mot "noeud" à la place de "sommet de triangle et/ou polygone" :

noeud $\equiv \{$ sommet de triangle et/ou polygone $\}$

### 1.1.3 Définitions préliminaires

**Ensemble des satellites $\Lambda(k)$**

Soit $\vec{\varphi}_k$ le $k$ème noeud constituant une surface $S$. Nous appellerons **ensemble des satellites** de

$\vec{\varphi}_k$ et nous noterons $\Lambda(k)$ l'ensemble des noeuds $\vec{\varphi}_j$ différents de $\vec{\varphi}_k$ tels que ( $\vec{\varphi}_j$ , $\vec{\varphi}_k$ ) soit un côté de triangle ou de polygone. En d'autres termes, l'ensemble $\Lambda(k)$ est tel que :

$$\left| \begin{array}{l} 1) \quad \Lambda(k) = \{\vec{\varphi}_{j_1}, \ldots, \vec{\varphi}_{j_{n_k}}\} \\ 2) \quad \vec{\varphi}_j \in \Lambda(k) \Longleftrightarrow \left\{ \begin{array}{l} \vec{\varphi}_j \text{ et } \vec{\varphi}_k \text{ sont les extrémités d'un} \\ \text{même côté de triangle ou de poly-} \\ \text{gone} \end{array} \right. \end{array} \right.$$

Comme indiqué dans la relation ci-dessus, nous conviendrons de noter $n_k$ le nombre de satellites rattachés

au noeud $\vec{\varphi}_k$ .

Cette notion d'orbite introduite dans les articles [6] et [7] n'a jusqu'à présent pas fait l'objet de procédé de codage particulier; une partie importante de l'invention consiste à proposer un procédé de codage pour $\Lambda(k)$.

**Voisinage** $N(k)$

Soit $\vec{\varphi}_k$ le $k$ème noeud d'une surface $S$. Nous appellerons **voisinage** de $\vec{\varphi}_k$ et nous noterons $N(k)$ l'ensemble des noeuds constitués par la réunion de $\vec{\varphi}_k$ et de ses satellites. En d'autres termes, on a :

$$N(k) = \{\ \vec{\varphi}_k\ \} \cup \Lambda\ (k)$$

Nous appellerons **voisin** du noeud $\vec{\varphi}_k$ tout noeud appartenant à l'ensemble $N(k)$. En d'autres termes, on a :

$$\vec{\varphi}_j \in N(k) \Longleftrightarrow \{\vec{\varphi}_j = \text{Voisin de } \vec{\varphi}_k\}$$

Cette notion de voisinage introduite dans les articles [7] et [8] n'a jusqu'à présent pas fait l'objet de procédé de codage particulier; une partie importante de l'invention consiste à proposer un procédé de codage pour $N(k)$.

**Atome** $A(k)$ **et noyau associé**

Soit $\vec{\varphi}_k$ le $k$ème noeud d'une surface $S$. Nous appellerons **atome** de **noyau** $\vec{\varphi}_k$ et nous noterons $A(k)$ le couple ( $\vec{\varphi}_k$ , $\Lambda^*(k)$) constitué par la juxtaposition (et non la réunion) :

- du noeud $\vec{\varphi}_k$
- de l'ensemble $\Lambda^*(k)$ des adresses ou des codes $\{A^*(j_1),..., A^*(j_{n_k})\}$ permettant de retrouver dans une mémoire l'ensemble des atomes $\{A(j_1),..., A\ (j_{n_k})\}$ correspondants dont les noyaux sont les satellites de

$\vec{\varphi}_k$

En d'autres termes, on a :

$$\left|\ \begin{aligned} A(k) \quad &= \quad (\vec{\varphi}_k, \Lambda^*(k)) = (\vec{\varphi}_k, \{A^*(j_1), \ldots, A^*(j_{n_k})\}) \\[2em] \text{avec} \quad : &\begin{cases} n_k \quad = \quad \text{nombre de satellites de } \vec{\varphi}_k \\[1em] A^*(j) \quad = \quad \text{Adresse ou code d'accès de l'atome} \\ \qquad\qquad A(j) \text{ correspondant au noyau du } j^{\text{ème}} \\ \qquad\qquad \text{satellite de } \vec{\varphi}_k \end{cases} \end{aligned}\right.$$

Cette notion d'atome introduite dans l'article [6] n'a jusqu'à présent pas fait l'objet de procédé de codage particulier; une partie importante de l'invention consiste à proposer un procédé de codage pour $A(k)$.

**Contraintes et types de contraintes**

Nous appellerons contrainte relative à un atome $A(k)$, toute condition relative à la position du noeud $\vec{\varphi}_k$ correspondant au noyau de cet atome. Par exemple, en géologie on peut citer les contraintes suivantes qui sont d'une importance capitale pour l'ajustement d'une surface aux données sures ou approchées observées ou mesurées par des procédés manuels ou géophysiques :

- Les coordonnées $(\varphi_k^x, \varphi_k^y, \varphi_k^z)$ du noeud $\vec{\varphi}_k$ sont connues de façon exacte. Une telle contrainte est dite de type **noeud de contrôle** et est utilisée en géologie par exemple pour coder l'intersection de la surface modélisée avec un forage.

- Les coordonnées ($\varphi_k^x$, $\varphi_k^y$, $\varphi_k^z$) du noeud $\vec{\varphi}_k$ sont connues de façon approchée avec un coefficient de certitude donné. Une telle contrainte est dite de type **noeud de contrôle flou** et est utilisée pour coder la position approximative d'un noeud de la surface modélisée supposée mesurée de façon approximative par un procédé donné. Par exemple,
  - en géologie, les données sismiques sont de ce type,
  - en biologie, les données de coupes d'organes observées sous microscope ou par échographie ou encore par scanner sont de ce type.

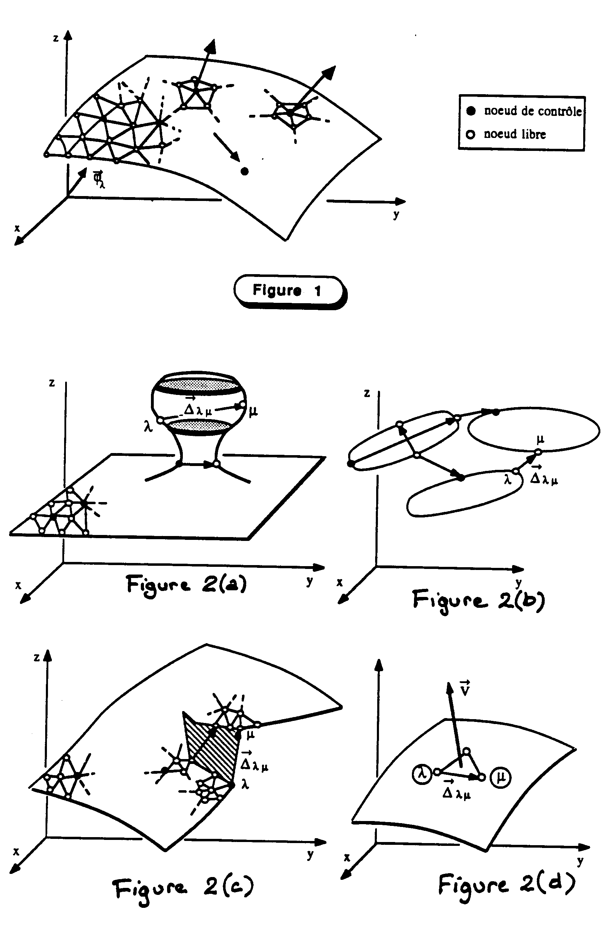

- Le vecteur ( $\vec{\varphi}_\lambda$ - $\vec{\varphi}_k$ ) joignant le noeud $\vec{\varphi}_k$ à un autre noeud $\vec{\varphi}_\lambda$ est connu de façon approchée avec un coefficient de certitude donné. Une telle contrainte est dite de type **lien vectoriel flou** et est utilisée en géologie par exemple pour coder le rejet d'une faille, le noeud $\vec{\varphi}_k$ étant sur un bord de la faille et le noeud $\vec{\varphi}_\lambda$ étant alors sur le bord opposé (voir Figure 2).

- Le vecteur $\vec{V}$ orthogonal au plan tangent à la surface $S$ au point correspondant au noeud $\vec{\varphi}_k$ est connu de façon approchée avec un coefficient de certitude donné. Une telle contrainte est dite de type **normale floue** et est utilisée en géologie par exemple pour coder le pendage des couches mesuré par des procédés manuels ou géophysiques (pendagemétrie).

- etc ...

Les coefficients de certitude mentionnés ci-dessus sont des nombres positifs dont la valeur est censée représenter la confiance que l'on peut avoir "a priori" dans les données qui lui sont attachées. La prise en compte des contraintes floues présentées ci-dessus constitue l'une des caractéristiques de l'invention et est présentée en détail dans l'annexe (voir annexe - section 2.5 et 2.7.6.).

**Convention**

Afin de simplifier les notations, on convient d'identifier les numéros des noeuds d'une surface $S$ avec les noeuds eux-mêmes. Il s'ensuit que l'on a :

- $\Lambda(k)$ = ensemble des satellites { $\vec{\varphi}_{j_1}$ ,..., $\vec{\varphi}_{j_{n_k}}$ } de $\vec{\varphi}_k$ ou ensemble des numéros $\{j_1,...j_{n_k}\}$ correspondants

- $N(k)$ = ensemble des voisins { $\vec{\varphi}_k$ , $\vec{\varphi}_{j_1}$ ,..., $\vec{\varphi}_{j_{n_k}}$ } de $\vec{\varphi}_k$ ou ensemble des numéros $\{k, j_1,...j_{n_k}\}$ correspondants

### 1.1.4 Etat de l'art

**Les méthodes classiques**

On trouvera dans la référence [7] une description très complète de l'état de l'art concernant la modélisation de surfaces complexes. En particulier, dans cet article, on expose les raisons pour lesquelles les méthodes classiques utilisées en cartographie automatique et en Conception Assistée par Ordinateur (CAO) ne sont pas adaptées à la modélisation de telles surfaces comme on aurait pu le croire a priori. A la fin de cet article [7], on introduit brièvement les fondements de la méthode DSI (quelquefois appelée DSI/DSA) permettant de surmonter les difficultés inhérente aux méthodes classiques.

**La méthode DSI**

Cette méthode est basée sur la notion de "rugosité" d'une surface $S$ composée de facettes triangulaires et/ou polygonales. Tout d'abord, la notion de rugosité $R(\vec{\varphi} \mid k)$ au noeud $\vec{\varphi}_k$ d'une surface $S$ est définie à l'aide de la formule suivante (Voir ref. [7], [8] et Annexe) :

$$R(\vec{\varphi}|k) \;=\; \|\sum_{\alpha \in N(k)} v^{\alpha}(k) \cdot \vec{\varphi}_{\alpha}\|^2$$

Les coefficient positifs ou nuls $\{ v^{\alpha}(k)\}$ intervenant dans la formule ci-dessus sont des pondérateurs choisis par l'utilisateur chargé de modéliser la surface. Parmi l'infinité de choix possibles, l'un des plus intéressants appellé "pondération harmonique" consiste à poser :

$$\vec{\varphi}$$

Les rugosités locales ainsi définies sont ensuite combinées en une rugosité globale $R(\vec{\varphi})$ définie comme suit :

$$\vec{\varphi}$$

Les coefficients positifs ou nuls $\mu(k)$ intervenant dans la formule ci-dessus servent à moduler la contribution des rugosités locales $R(\vec{\varphi}\,|\,k)$ à la rugosité globale $R(\vec{\varphi})$. Si l'on décide par exemple d'utiliser une pondération uniforme, il suffit de poser :

$$\mu(k) \;=\; 1 \; \forall \, k$$

Parmi les autres choix possibles pour les coefficients $\mu(k)$, on peut décider d'utiliser par exemple les pondérateurs suivants où $m$ est une constante donnée :

$$\mu(k) = \left\{ \begin{array}{ll} 1 & \text{si } \vec{\varphi}_k \text{ est un point de donnée} \\ m > 1 & \text{si } \vec{\varphi}_k \text{ n'est pas un point de donnée} \end{array} \right.$$

Le principe de la méthode DSI consiste à ajuster la position de chacun des noeuds $\vec{\varphi}_k$ de la surface $S$ modélisée de telle sorte que celle-ci soit la plus lisse possible tout en respectant les données et contraintes régissant sa forme. L'article [8] mentionné en bibliographie est consacré à une présentation mathématique approfondie de la méthode DSI et propose une technique de minimisation de la rugosité globale respectant les données et contraintes.

**Version interactive de la méthode DSI**

La méthode de minimisation de la rugosité globale de la méthode DSI proposée dans l'article [8] est mal adaptée à une utilisation interactive sur calculateur électronique (ordinateur ou microprocesseur spécialisé), c'est pourquoi nous proposons en annexe une autre technique de minimisation originale sur laquelle est basée l'invention. Ainsi qu'il a été dit précédemment, l'objet de l'invention est précisément de proposer un procédé de codage et un procédé d'ajustement de la surface $S$ suivant cette nouvelle version de la méthode DSI.

**1.2 Exemples d'images d'objets codés et ajustés par notre procédé**

**1.2.1 Exemple d'image de surface géologique obtenue par notre procédé**

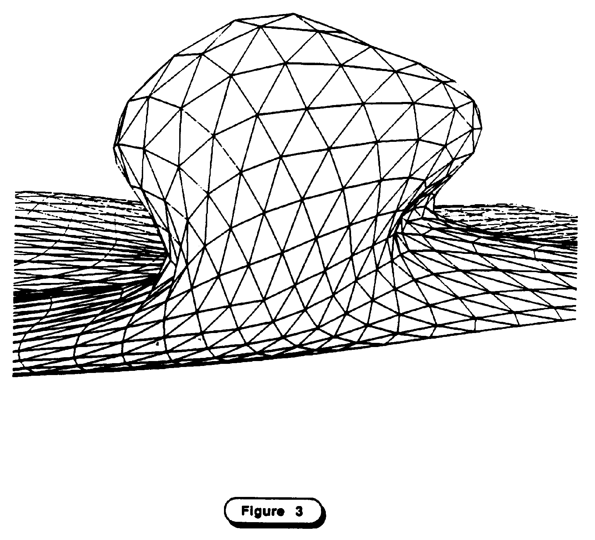

La recherche du pétrole est généralement effectuée de façon indirecte en ce sens que plutôt que de rechercher le pétrole, on cherche des couches géologiques réservoir suceptibles de le contenir ou de le capturer; par exemple, la déformation des couches de sel donne naissance à ce que l'on appelle des "dômes de sel" qui sont considérés comme générant d'excellents pièges à pétrole. Pour pouvoir localiser avec précision la position du pétrole il est nécessaire de connaître la forme exacte des couches "pièges" et malheureusement dans de nombreux cas il n'existait jusqu'à ce jour aucun procédé pratique permettant de les modéliser de façon satisfaisante; le cas des dômes de sel est à ce titre considéré comme l'un des plus complexes et pour prouver l'efficacité de notre procédé de codage et ajustement nous présentons sur la Figure 3 l'image d'une telle surface obtenue avec celui-ci.

### 1.2.2 Exemple d'image de surface biologique obtenue par notre procédé

Les procédés d'acquisition de données tels que scanner ou échographie utilisés en médecine sont mathématiquement identiques aux procédés d'acquisition de données sismiques utilisés en prospection pétrolière. Par exemple, l'échographie est identique à la sismique réflexion et le fonctionnement des scanners est basé sur la tomographie tout comme la tomographie sismique. Dès lors, il n'est pas étonnant que notre procédé de codage et ajustement des surfaces soit également utilisable pour modéliser la peau d'organes biologiques et nous proposons sur la Figure 4 le modèle du cerveau d'un embryon humain de 7 millimètres de long obtenu à l'aide de notre procédé de codage et d'ajustement. Il est à noter qu'un aussi petit organe (le cerveau fait 1 millimètre de long) ne pouvait jusqu'à présent être observé que sous forme de coupes microscopiques.

### 1.3 Procédé de codage d'une surface $S$

### 1.3.1 Notion de mémoire et d'adresse de mémoire

Nous appellerons « mémoire" tout dispositif de calculatrice électronique qui enregistre des données, des codes ou résultats partiels qu'il restitue au moment opportun pour les faire intervenir dans la suite des opérations. De plus, nous appellerons "adresse" d'une mémoire, tout code permettant de retrouver l'emplacement physique de la mémoire à l'intérieur de l'ordinateur.

### 1.3.2 Codage d'un atome $A(k)$

Nous proposons de coder et stocker chaque atome $A(k)$ dans un ensemble de mémoires à l'aide du procédé suivant ou l'une de ses variantes :

### Procédé de codage

Comme le suggère la Figure 5, dans une zone mémoire contiguë commençant à l'adresse $A^*(k)$, on stocke de façon consécutive les informations suivantes relatives à l'atome $A(k)$ correspondant au noeud $\vec{\varphi}_k$ :

1. **noyau =**

- soit les coordonnées $(\varphi_k^x, \varphi_k^y, \varphi_k^z)$ du noeud $\vec{\varphi}_k$
- soit l'adresse ou le code ou toute autre information permettant de retrouver les coordonnées $(\varphi_k^x, \varphi_k^y,$ $\varphi_k^z)$ du noeud $\vec{\varphi}_k$

2. **nb_sat =**

- soit le nombre $n_k$ de satellites liés au noeud $\vec{\varphi}_k$
- soit l'adresse ou le code ou toute autre information permettant de retrouver le nombre $n_k$ de satellites liés au noeud $\vec{\varphi}_k$

3. **sat =**
Adresse d'une zone mémoire contenant les adresses ou les codes d'accès $\{A^*(j_1),..., A^*(j_{n_k})\}$ permettant de retrouver les $n_k$ atomes $\{A(j_1),..., A(j_{n_k})\}$ dont les noyaux $\{\vec{\varphi}_{j_1},..., \vec{\varphi}_{j_{n_k}}\}$ sont les satellites du noeud $\vec{\varphi}_k$

4. **const =**

Codage de l'ensemble des contraintes (cf section 1.1.3) pouvant affecter le noeud $\vec{\varphi}_k$. Ce codage sera présenté en détails dans la section 1.3.3.

5. **info =**
Zone mémoire de taille variable contenant zéro, une ou plusieurs informations complémentaires concernant l'atome $A(k)$. Ces informations sont spécifiques à l'application particulière envisagée; par exemple en prospection pétrolière, si $A(k)$ est un atome correspondant à la surface séparant deux couches $C_1$ et $C_2$, alors il peut être intéressant de stocker dans le champ **info** des informations telles que :

- liste des propriétés physiques (porosités, vitesse des ondes sismiques, ...) de la couche $C_1$ au noeud $\vec{\varphi}_k$ correspondant à l'atome $A(k)$
- liste des propriétés physiques (porosités, vitesse des ondes sismiques, ...) de la couche $C_2$ au noeud $\vec{\varphi}_k$ correspondant à l'atome $A(k)$
- liste des propriétés géologiques (faciès géologique, présence de pétrole, ...) de la couche $C_1$ au noeud $\vec{\varphi}_k$ correspondant à l'atome $A(k)$
- liste des propriétés géologiques (faciès géologique, présence de pétrole, ...) de la couche $C_2$ au noeud $\vec{\varphi}_k$ correspondant à l'atome $A(k)$
- etc ..

**Variante 1**

La zone mémoire dans laquelle sont stockées les adresses ou les codes d'accès $\{A^*(j_1),...,A^*(j_{n_k})\}$ permettant de retrouver les $n_k$ atomes $\{A(j_1),..., A(j_{n_k})\}$ dont les noyaux sont les satellites $\{\vec{\varphi}_{j_1} ,..., \vec{\varphi}_{j_{n_k}} \}$ du noeud $\vec{\varphi}_k$ peut être structurée de deux façons :

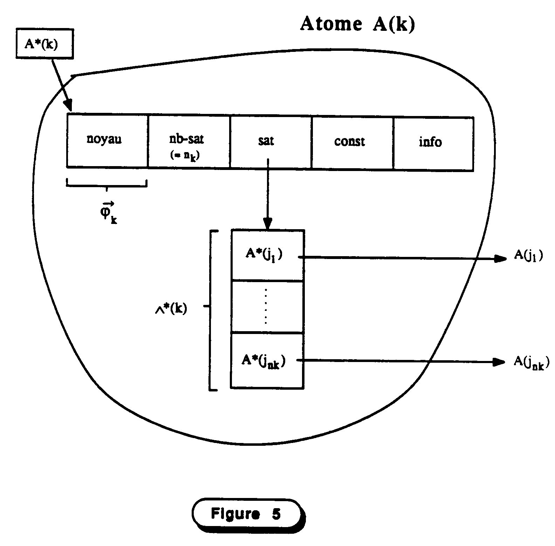

1. La première façon illustrée par la Figure 6 consiste à utiliser un tableau de $n_k$ mémoires consécutives contenant les adresses $\{A^*(j_1),..., A^*(j_{n_k})\}$

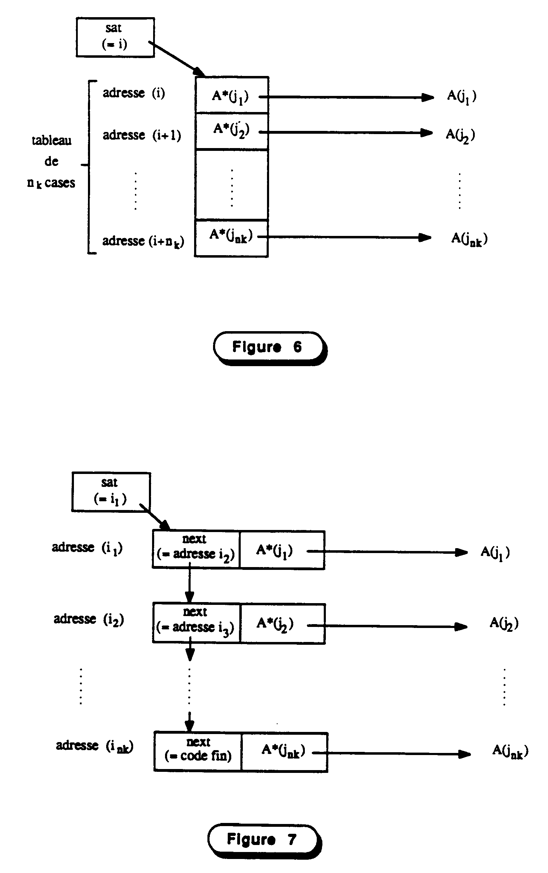

2. La seconde façon illustrée par la Figure 7 consiste à utiliser $n_k$ tableaux comportant chacun 2 mémoires consécutives telles que pour le $\alpha^{ème}$ de ces tableaux :
- la première mémoire contienne l'adresse $A^*(j_\alpha)$ de l'atome correspondant au $\alpha^{ème}$ satellite de l'atome $A(k)$
- la seconde mémoire contienne l'adresse du $(\alpha+1)^{ème}$ tableau. Si $\alpha$ est égal au nombre $n_k$ de satellites (**nb_sat**=$n_k$), alors cette seconde mémoire est soit inutilisée, soit contient un code indiquant que l'on a atteint le dernier satellite de $A(k)$

**Variante 2**

Il est possible de ne pas stocker le champ **nb_sat** mais dans ce cas, si la liste des adresses ou codes d'accès $\{A^*(j_1),..., A^*(j_{n_k})\}$ est codée sous forme d'un seul tableau (voir variante 1), il faut rajouter un emplacement mémoire $A^*(n_k + 1)$ dans lequel on place un code indiquant que la liste $\Lambda^*(k)$ est terminée.

**Variante 3**

Le champ **sat** peut être choisi suffisamment grand de façon à y stocker directement les adresses ou codes d'accès $\{A^*(j_1),..., A^*(j_{n_k})\}$

**Variante 4**

Un grand nombre de variantes équivalentes sont possibles suivant la taille du champ **info** et de la façon dont on redécoupe celui-ci. Il est toutefois bien entendu que ces variantes n'ont aucune importance pour l'utilisation de l'atome $A(k)$ en connection avec l'algorithme d'ajustement basé sur la méthode DSI décrite en annexe.

**Variante 5**

On peut rajouter d'autres champs à l'atome $A(k)$ décrit ci-dessus mais ceux-ci n'ont aucune importance pour l'utilisation de l'atome $A(k)$ en connection avec l'algorithme d'ajustement basé sur la méthode DSI décrit en annexe.

**Variante 6**

Quelles que soient les variantes, la seule chose importante pour l'implémentation efficace de l'algorithme DSI est la suivante :

Ayant l'adresse ou le code d'accès $A^*(k)$ d'un atome $A(k)$, il est nécessaire d'accéder de la façon la plus directe possible aux atomes dont les noyaux sont les satellites du noyau de $A(k)$.

L'objet essentiel du procédé de codage de $A(k)$ dans une mémoire décrit ci-dessus ainsi que toutes ses variantes est précisément de permettre l'accès direct aux satellites.

## 1.3.3 Codage des contraintes

Chaque contrainte relative à un atome $A(k)$ sera stockée dans une zone mémoire spécifique dont la description est proposée dans ce qui suit :

**Procédé de stockage d'une contrainte**

Le codage d'une contrainte particulière relative à un atome donné est effectué dans une zone mémoire comprenant les deux champs **contrainte** et **next** comme l'indique la Figure 8. Ces deux champs sont utilisés de la façon suivante :

- **contrainte =**
  Zone mémoire contenant :
  - soit les informations relatives à la contrainte codée (voir section plus bas)
  - soit l'adresse ou le code d'accès d'une zone mémoire contenant effectivement les informations relatives à la contrainte codée

Parmi les informations relatives à la contrainte codée, doit toujours figurer un code permettant de reconnaître le **type** de la contrainte codée (cf section 1.1.3).

- **next =**
  zone mémoire contenant :
  - soit un code indiquant que la contrainte considérée est la dernière des contraintes affectant l'atome $A(k)$.
  - soit l'adresse d'une zone mémoire contenant la contrainte suivante (voir section suivante).

**Procédé de stockage de toutes les contraintes affectant un atome**

Le codage d'un atome $A(k)$ décrit dans la section 1.3.2 contient un champ **const** que nous utiliserons pour stocker :

- soit un code indiquant que l'atome $A(k)$ n'est affecté par aucune contrainte
- soit l'adresse d'une zone mémoire contenant la première contrainte affectant l'atome $A(k)$

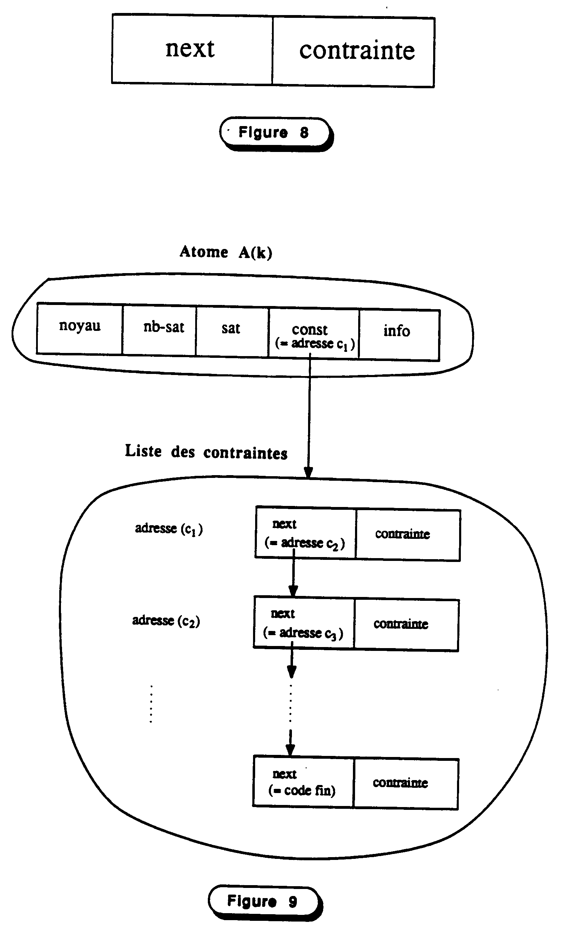

Si l'adresse de la $n^{\text{ème}}$ contrainte relative à l'atome $A(k)$ est stockée dans le champ **next** de la $(n-1)^{\text{ème}}$ contrainte ($n > 1$), alors, comme le suggère la Figure 9, on est assuré que le procédé de codage décrit permet d'attacher à l'atome $A(k)$ toutes les contraintes qui lui sont relatives. Ce mode de codage permet de rajouter ou de supprimer facilement des contraintes sans remettre en question l'organisation des autres données.

**Exemples d'informations relatives à une contrainte donnée**

Dans la section 1.1.3 nous avons présenté à titre d'exemple trois types de contraintes très importants pour le codage et l'ajustement des surfaces complexes rencontrées en biologie ou géologie. A chacun de ces types de contraintes correspondent des informations qui doivent être associées au champ **contrainte** décrit au paragraphe précédent et schématisé sur la Figure 9:

- Pour une contrainte de type **noeud de contrôle**, en plus du type, il est nécessaire de stocker au minimum les données suivantes :

  - les coordonnées $(\varphi_k^x, \varphi_k^y, \varphi_k^z)$ approchées du noeud $\vec{\varphi}_k$ correspondant au noyau de l'atome $A(k)$ auquel est attachée la contrainte
  - le coefficient de certitude (nombre positif) mesurant la confiance que l'on peut avoir dans les coordonnées $(\varphi_k^x, \varphi_k^y, \varphi_k^z)$.

- Pour une contrainte de type **lien vectoriel**, en plus du type, il est nécessaire de stocker au minimum les données suivantes :

- l'adresse du noeud $\vec{\varphi}_\lambda$ auquel est lié le noeud $\vec{\varphi}_k$ correspondant au noyau de l'atome $A(k)$ auquel est attachée la contrainte

- les trois composantes approchées du vecteur ( $\vec{\varphi}_\lambda$ - $\vec{\varphi}_k$ ) joignant le noeud $\vec{\varphi}_k$ à au noeud

  $\vec{\varphi}_\lambda$

- le coefficient de certitude (nombre positif) mesurant la confiance que l'on peut avoir dans les composantes approximatives du vecteur ( $\vec{\varphi}_\lambda$ - $\vec{\varphi}_k$ ).

- Pour une contrainte de type **normale,** en plus du type, il est nécessaire de stocker au minimum les données suivantes :

- les trois composantes approchées du vecteur $\vec{V}$ orthogonal à la surface $S$ au noeud $\vec{\varphi}_k$ correspondant au noyau de l'atome $A(k)$ auquel est attachée la contrainte
- le coefficient de certitude (nombre positif) mesurant la confiance que l'on peut avoir dans les composantes approximatives du vecteur $\vec{V}$ .

La présentation détaillée de ces trois types de contraintes est proposée dans les sections 2.5 et 2.7.6 de l'annexe.

**Variante**

Bien que l'on n'ait parlé dans ce qui précède que d'un seul coefficient de certitude pour les composantes en $x$, $y$ et $z$ de chaque contrainte, on pourra affecter à ces 3 composantes 3 coefficients de certitude différents si nécessaire.

**1.3.4 Codage d'une surface $S$**

Afin d'optimiser la programmation de la variante de la méthode DSI présentée en annexe, nous proposons de coder une surface $S$ composée de facettes triangulaires et/ou polygonales sous forme d'un ensemble de N atomes dont les noyaux (noeuds) sont les sommets de ces triangles et/ou polygones. Les données et codes correspondant à chacun de ces atomes $\{A(1),...,A(N)\}$ peuvent être stockés :

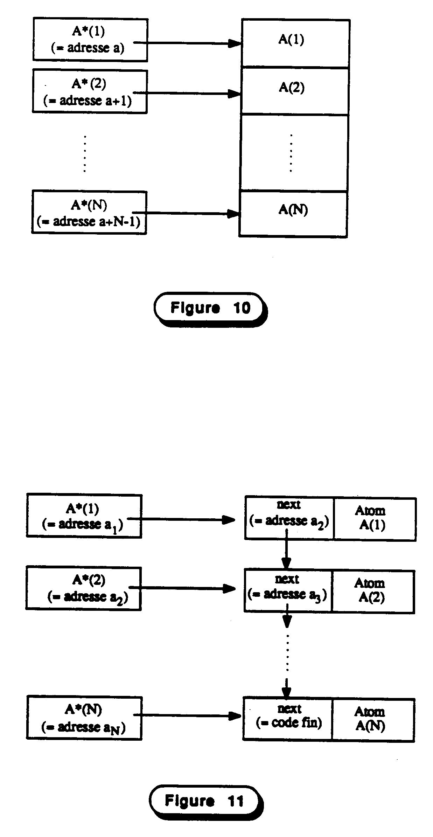

- soit dans un tableau comme le suggère la Figure 10
- soit dans le champ **atom** de zones mémoires chaînées entre elles par leur champ **next** comme le suggère la Figure 11.Dans ce cas, le champ **next** associé à l'atome numéro $k$ doit contenir l'adresse ou le code d'accès de l'atome suivant numéroté ($k + 1$); le champ **next** associé au dernier atome $A(N)$ doit contenir un code indiquant que le dernier atome a été atteint.

La deuxième solution est la plus intéressante si l'on souhaite pouvoir ajouter ou supprimer facilement des atomes, par contre elle consomme d'avantage de place mémoire que la première.

**1.3.5 Variantes**

Pour des commodités de présentation, les zones mémoires utilisées pour coder des informations ont été découpées en champs et à chacun de ces champs a été attribué :

- un nom propre
- une place dans la zone mémoire correspondante

Il est bien évident que le procédé de codage décrit dans la présente demande de brevet ne dépend pas des noms ni de l'ordre de ces champs à l'intérieur des zones mémoire utilisées pour le codage. Par ailleurs, il est toujours possible de rajouter des champs à des fins spécifiques à certaines applications sans que cela modifie le procédé de stockage.

**1.4 Ajustement d'une surface $S$**

**1.4.1 Introduction**

Etant donné une surface $S$ codée suivant le procédé décrit dans la section 1.3, nous nous proposons dans ce qui suit de décrire un procédé basé sur ce codage et permettant d'ajuster de façon optimale les coordonnées de certains des noeuds { $\vec{\varphi}_1$ ,...., $\vec{\varphi}_N$ } et ceci de sorte que la surface $S$ :

- soit la plus lisse possible au sens du critère de rugosité globale de la méthode DSI (voir références [7], [8] et annexe ainsi que la section 1.1.4)
- respecte le mieux possible les informations et contraintes connues avec plus ou moins de certitude concernant la forme de cette surface ( voir sections 1.1.1 et 1.1.3)

Pour ce faire, on utilisera une variante originale de la méthode DSI présentée en annexe, variante spécialement conçue pour utiliser le codage décrit dans la section 1.3. Compte tenu des informations disponibles observées ou mesurées, cette méthode ajuste successivement et de façon itérative les trois coordonnées ($\varphi_\alpha^x$,

$\varphi_\alpha^y$, $\varphi_\alpha^z$) de chaque noeud $\vec{\varphi}_\alpha$ non fixé suivant une formule du type :

$$\begin{aligned}
\text{nouvelle valeur de } \varphi_\alpha^x &= f_\alpha^x( \text{ anciennes valeurs de } \varphi_1^x,\ldots,\varphi_N^x) \\
\text{nouvelle valeur de } \varphi_\alpha^y &= f_\alpha^y( \text{ anciennes valeurs de } \varphi_1^y,\ldots,\varphi_N^y) \\
\text{nouvelle valeur de } \varphi_\alpha^z &= f_\alpha^z( \text{ anciennes valeurs de } \varphi_1^z,\ldots,\varphi_N^z)
\end{aligned}$$

Plus précisément, si l'on se reporte à l'annexe présentant la variante utilisée de la méthode DSI, ces fonctions d'ajustement $f_\alpha^x()$, $f_\alpha^y()$ et $f_\alpha^z()$ sont déduites de la forme locale des équations DSI au point numéro $\alpha$ et ont la forme suivante (voir sections 2.4.1, 2.4.2 et 2.7.3 de l'annexe) :

$$
\begin{aligned}
f_\alpha^x(\cdots) &= -\frac{1}{M_\alpha^x} \cdot \left\{ \sum_{k \in N(\alpha)} \{\mu(k)\, v^\alpha(k) \cdot \sum_{\substack{\beta \in N(k) \\ \beta \neq \alpha}} v^\beta(k) \cdot \varphi_\beta^x\} \right. \\
&\qquad \left. + \sum_i \{\Gamma_i^{x\alpha} - Q_i^{x\alpha}\} \right\} \\[2ex]
f_\alpha^y(\cdots) &= -\frac{1}{M_\alpha^y} \cdot \left\{ \sum_{k \in N(\alpha)} \{\mu(k)\, v^\alpha(k) \cdot \sum_{\substack{\beta \in N(k) \\ \beta \neq \alpha}} v^\beta(k) \cdot \varphi_\beta^y\} \right. \\
&\qquad \left. + \sum_i \{\Gamma_i^{y\alpha} - Q_i^{y\alpha}\} \right\} \\[2ex]
f_\alpha^z(\cdots) &= -\frac{1}{M_\alpha^z} \cdot \left\{ \sum_{k \in N(\alpha)} \{\mu(k)\, v^\alpha(k) \cdot \sum_{\substack{\beta \in N(k) \\ \beta \neq \alpha}} v^\beta(k) \cdot \varphi_\beta^z\} \right. \\
&\qquad \left. + \sum_i \{\Gamma_i^{z\alpha} - Q_i^{z\alpha}\} \right\}
\end{aligned}
$$

$$\text{avec :} \begin{cases} M_\alpha^{\star x} = M_\alpha + \sum_i \gamma_i^{x\alpha} \\[2mm] M_\alpha^{\star y} = M_\alpha + \sum_i \gamma_i^{y\alpha} \\[2mm] M_\alpha^{\star z} = M_\alpha + \sum_i \gamma_i^{z\alpha} \\[2mm] M_\alpha = \sum_{k \in N(\alpha)} \mu(k)\,(v^\alpha(k))^2 \end{cases}$$

La nature et le rôle des coefficients de pondération $\{v^\alpha(k)\}$ et $\mu(k)$ ont été indiqués section 1.1.4 et sont détaillés dans l'article [8] ainsi qu'en annexe. Les valeurs des termes $\{\Gamma_i^{x\alpha}, \Gamma_i^{y\alpha}, \Gamma_i^{z\alpha}\}$, $\{\gamma_i^{x\alpha}, \gamma_i^{y\alpha}, \gamma_i^{x\alpha}\}$ et des termes

$\{Q_i^{x\alpha}, Q_i^{y\alpha}, Q_i^{z\alpha}\}$ dépendent des types et des valeurs des contraintes affectant le noeud $\vec{\varphi}_\alpha$ et leur formulation exacte est présentée en annexe.

Dans le cas particulier où les coefficients de pondération $\{v^\alpha(k)\}$ correspondent à la pondération harmonique présentée section 1.1.4 et en annexe, les fonctions de pondération $f_\alpha^x()$, $f_\alpha^y()$ et $f_\alpha^z()$ se simplifient et prennent la forme suivante :

$$f_\alpha^x(\cdots) = \frac{1}{M_\alpha^{\star x}} \cdot \Bigg[ \quad \mu(\alpha) \cdot |\Lambda(\alpha)| \cdot \Bigg( \sum_{k \in \Lambda(\alpha)} \varphi_k^x \Bigg)$$
$$- \sum_{k \in \Lambda(\alpha)} \mu(k) \cdot \Bigg\{ \Bigg( \sum_{\substack{\beta \in \Lambda(k) \\ \beta \neq \alpha}} \varphi_\beta^x \Bigg) - |\Lambda(k)| \cdot \varphi_k^x \Bigg\}$$
$$- \sum_i \{ \Gamma_i^{x\alpha} - Q_i^{x\alpha} \} \quad \Bigg]$$

$$f_\alpha^y(\cdots) = \frac{1}{M_\alpha^{\star y}} \cdot \Bigg[ \quad \mu(\alpha) \cdot |\Lambda(\alpha)| \cdot \Bigg( \sum_{k \in \Lambda(\alpha)} \varphi_k^y \Bigg)$$
$$- \sum_{k \in \Lambda(\alpha)} \mu(k) \cdot \Bigg\{ \Bigg( \sum_{\substack{\beta \in \Lambda(k) \\ \beta \neq \alpha}} \varphi_\beta^y \Bigg) - |\Lambda(k)| \cdot \varphi_k^y \Bigg\}$$
$$- \sum_i \{ \Gamma_i^{y\alpha} - Q_i^{y\alpha} \} \quad \Bigg]$$

$$f_\alpha^z(\cdots) = \frac{1}{M_\alpha^{\star z}} \cdot \Bigg[ \quad \mu(\alpha) \cdot |\Lambda(\alpha)| \cdot \Bigg( \sum_{k \in \Lambda(\alpha)} \varphi_k^z \Bigg)$$
$$- \sum_{k \in \Lambda(\alpha)} \mu(k) \cdot \Bigg\{ \Bigg( \sum_{\substack{\beta \in \Lambda(k) \\ \beta \neq \alpha}} \varphi_\beta^z \Bigg) - |\Lambda(k)| \cdot \varphi_k^z \Bigg\}$$
$$- \sum_i \{ \Gamma_i^{z\alpha} - Q_i^{z\alpha} \} \quad \Bigg]$$

$$\text{avec :} \begin{cases} M_\alpha^{\star x} = M_\alpha + \sum_i \gamma_i^{x\alpha} \\ M_\alpha^{\star y} = M_\alpha + \sum_i \gamma_i^{y\alpha} \\ M_\alpha^{\star z} = M_\alpha + \sum_i \gamma_i^{z\alpha} \\ M_\alpha = \mu(\alpha) \cdot |\Lambda(\alpha)|^2 + \sum_{k \in \Lambda(\alpha)} \mu(k) \end{cases}$$

Nous proposons dans ce qui suit un procédé de calcul de ces fonctions basé sur le codage présenté au paragraphe 1.3:

### 1.4.2 Notations

Afin de simplifier la description des procédés de calculs présentés dans ce qui suit, il est commode d'introduire les notations et définitions suivantes :

- Par définition, nous appellerons **bloc d'opérations**, toute liste d'opérations précèdée d'une accolade ouvrante "{" et terminée par une accolade fermante "}". Les opérations figurant dans un bloc d'opérations peuvent être des opérations élémentaires et/ou d'autres blocs d'opérations.
- Par définition, nous appellerons mémoire de travail toute mémoire utilisée pour stocker des résultats intermédiaires au cours d'un procédé de calcul.
- Par définition, nous appellerons "chargement" de l'expression $e$ dans une mémoire $m$ l'opération consistant à évaluer la valeur de $e$ puis à ranger le résultat dans la mémoire $m$. Par convention, une telle opération sera notée de la façon suivante :

$$m \leftarrow e$$

- Pour toute mémoire $m$ utilisée pour stocker une valeur numérique, nous appellerons "incrémentation" de $m$ par l'incrément $i$ l'opération consistant à calculer la valeur $m + i$ puis à ranger le résultat dans la mémoire $m$. Par convention, une telle opération sera notée de la façon suivante :

$$m \leftarrow m + i$$

Dans l'opération ci-dessus, l'incrément $i$ peut être :
- soit une constante
- soit le contenu d'une mémoire
- soit une expression arithmétique

### 1.4.3 Procédé d'ajustement dans le cas de pondérateurs harmoniques

Dans le cas où les coefficients $\{v^\alpha(k)\}$ sont les pondérateurs harmoniques présentés dans la section 1.1.4 et dans l'annexe, les valeurs $f'_\alpha$, $f''_\alpha$ et $f'''_\alpha$ des fonctions d'ajustement $f'_\alpha()$, $f''_\alpha()$ et $f'''_\alpha()$ relatives au noeud

$\vec{\varphi}_\alpha$ peuvent être calculées de façon efficace à l'aide du procédé suivant basé sur le codage de $S$ :

0) Allouer les mémoires de travail suivantes :

**14**

$$f_\alpha^x, f_\alpha^y, f_\alpha^z$$

$$\sigma_\varphi^x, \sigma_\varphi^y, \sigma_\varphi^z$$

$$s_\varphi^x, s_\varphi^y, s_\varphi^z$$

$$M_\alpha^{*x}, M_\alpha^{*y}, M_\alpha^{*z}, M_\alpha$$

$$\sigma_\mu$$

1) Effectuer les initialisations suivantes (l'ordre n'a aucune importance) :

$$f_\alpha^x = f_\alpha^y = f_\alpha^z = 0$$

$$\sigma_\varphi^x = \sigma_\varphi^y = \sigma_\varphi^z = 0$$

2) Déterminer le nombre $n_\alpha$ de satellites de $A(\alpha)$. Ce nombre peut être obtenu à partir du champ **nb_sat** de $A(\alpha)$

3) Soit $\Lambda(\alpha)$ l'ensemble des satellites de l'atome $A(\alpha)$ auxquels on accède par le champ **sat** de cet atome. Pour chaque satellite $k \in \Lambda(\alpha)$, répéter les opérations du bloc suivant :

{

3.1) Déterminer le nombre $n_k$ de satellites de $A(k)$. Ce nombre peut être obtenu à partir du champ **nb_sat** de $A(k)$

3.2) Déterminer les coordonnées $(\varphi_k^x, \varphi_k^y, \varphi_k^z)$ du noeud $\vec{\varphi}_k$ de l'atome $A(k)$. Ces coordonnées peuvent être obtenues à partir du champ **noeud** de $A(k)$.

3.3) A partir des informations contenues dans $A(k)$, calculer ou déterminer la valeur du pondérateur $\mu(k)$ (voir section 1.1.4 et annexe).

3.4) Effectuer les opérations suivantes (l'ordre n'a aucune importance).

$$\sigma_\mu \leftarrow \sigma_\mu + \mu(k)$$
$$\sigma_\varphi^x \leftarrow \sigma_\varphi^x + \varphi_k^x$$
$$\sigma_\varphi^y \leftarrow \sigma_\varphi^y + \varphi_k^y$$
$$\sigma_\varphi^z \leftarrow \sigma_\varphi^z + \varphi_k^z$$
$$s_\varphi^x = s_\varphi^y = s_\varphi^z = 0$$

3.5) Soit $\Lambda(k)$ l'ensemble des satellites de l'atome $A(k)$ auquel on accède par le champ **sat** de cet atome. Pour chaque satellite $\beta \in \Lambda(k)$, $\beta \neq \alpha$ effectuer les opérations du bloc suivant :

{

3.5.2) Déterminer les coordonnées actuelles $(\varphi_\beta^x, \varphi_\beta^y, \varphi_\beta^z)$ du noyau $\vec{\varphi}_\beta$ de l'atome $A(\beta)$. Ces coordonnées peuvent être obtenues à partir du champ **noeud** de $A(\beta)$.

3.5.3) Effectuer les 3 opérations suivantes (l'ordre n'a aucune importance) :

$$s_\varphi^x \leftarrow s_\varphi^x + \varphi_\beta^x$$
$$s_\varphi^y \leftarrow s_\varphi^y + \varphi_\beta^y$$
$$s_\varphi^z \leftarrow s_\varphi^z + \varphi_\beta^z$$

}

3.6) Effectuer les 3 opérations suivantes (l'ordre n'a aucune importance) :

$$\left|\begin{array}{l} f_\alpha^x \leftarrow f_\alpha^x + \mu(k) \times (s_\varphi^x - n_k \times \varphi_k^x) \\ f_\alpha^y \leftarrow f_\alpha^y + \mu(k) \times (s_\varphi^y - n_k \times \varphi_k^y) \\ f_\alpha^z \leftarrow f_\alpha^z + \mu(k) \times (s_\varphi^z - n_k \times \varphi_k^z) \end{array}\right.$$

}

4) Effectuer les opérations suivantes :

$$\left|\begin{array}{lll} M_\alpha & \leftarrow & \mu(\alpha) \times n_\alpha \times n_\alpha + \sigma_\mu \\ M_\alpha^{*x} & \leftarrow & M_\alpha \\ M_\alpha^{*y} & \leftarrow & M_\alpha \\ M_\alpha^{*z} & \leftarrow & M_\alpha \end{array}\right.$$

5) Soit $C(\alpha)$ l'ensemble des contraintes attachées à l'atome $A(\alpha)$ auquel on accède par le champ **const** de cet atome. Pour chaque contrainte $c \in C(\alpha)$ répéter les opérations du bloc suivant :

{

5.1) Déterminer le type de la contrainte $c$

5.2) En fonction du type de la contrainte $c$, effectuer les deux opérations suivantes :

5.2.1) Extraire les informations spécifiques de la contrainte $c$

5.2.2) En fonction des informations extraites en (5.2.1), calculer les valeurs des expressions suivantes (voir sections 2.5 et 2.7 de l'annexe) :

$$\left|\begin{array}{l} \Gamma_i^{x\alpha}, \Gamma_i^{y\alpha}, \Gamma_i^{z\alpha} \\ Q_i^{x\alpha}, Q_i^{y\alpha}, Q_i^{z\alpha} \\ \gamma_i^{x\alpha}, \gamma_i^{y\alpha}, \gamma_i^{z\alpha} \end{array}\right.$$

5.3) Effectuer les opérations suivantes (l'ordre n'a aucune importance) :

$$\left|\begin{array}{l} M_\alpha^{*x} \leftarrow M_\alpha^{*x} + \gamma_i^{x\alpha} \\ M_\alpha^{*y} \leftarrow M_\alpha^{*y} + \gamma_i^{y\alpha} \\ M_\alpha^{*z} \leftarrow M_\alpha^{*z} + \gamma_i^{z\alpha} \\ f_\alpha^x \leftarrow f_\alpha^x + (\Gamma_i^{x\alpha} - Q_i^{x\alpha}) \\ f_\alpha^y \leftarrow f_\alpha^y + (\Gamma_i^{y\alpha} - Q_i^{y\alpha}) \\ f_\alpha^z \leftarrow f_\alpha^z + (\Gamma_i^{z\alpha} - Q_i^{z\alpha}) \end{array}\right.$$

}

6) Effectuer les opérations suivantes (l'ordre n'a aucune importance) :

$$\left|\begin{array}{l} f_\alpha^x \leftarrow (\mu(\alpha) \times n_\alpha \times \sigma_\varphi^x - f_\alpha^x)/M_\alpha^{*x} \\ f_\alpha^y \leftarrow (\mu(\alpha) \times n_\alpha \times \sigma_\varphi^y - f_\alpha^y)/M_\alpha^{*y} \\ f_\alpha^z \leftarrow (\mu(\alpha) \times n_\alpha \times \sigma_\varphi^z - f_\alpha^z)/M_\alpha^{*z} \end{array}\right.$$

A la fin de ce procédé de calcul, les mémoires de travail $f_\alpha^x$, $f_\alpha^y$ et $f_\alpha^z$ contiennent les valeurs des fonctions

d'ajustement $f_\alpha^x()$, $f_\alpha^y()$ et $f_\alpha^z()$ utilisées pour ajuster les valeurs des coordonnées du noeud $\vec{\varphi}_\alpha$ :

$$\left| \begin{array}{l} \varphi_\alpha^x \leftarrow f_\alpha^x \\ \varphi_\alpha^y \leftarrow f_\alpha^y \\ \varphi_\alpha^z \leftarrow f_\alpha^z \end{array} \right.$$

Le procédé d'ajustement décrit ci-dessus ne doit être appliqué qu'aux noeuds dont la position est non fixée, c'est-à-dire aux noeuds qui ne sont pas des noeuds de contrôle (voir section 1.1.3).

## 1.4.4 Variantes

### Variante 1

La mise en oeuvre du procédé d'ajustement (basé sur la méthode DSI) présentée au paragraphe précédent dans le cadre de pondérateurs harmoniques peut facilement être adaptée dans le cas de pondérateurs quelconques. La structure globale du procédé reste la même, seule change l'incrémentation des différentes mémoires de travail allouées à l'étape (0); la façon d'incrémenter ces mémoires de travail dépend des pondérateurs choisis.

Par exemple, si nous choisissons les pondérateurs { $v^\alpha (k)$} suivants,

$$v^\alpha(k) = \left\{ \begin{array}{lll} -1 & \text{si} & \alpha = k \\ \frac{1}{|\Lambda(k)|} & \text{si} & \alpha \in \Lambda(k) \end{array} \right.$$

alors on vérifie facilement que les fonctions d'ajustement associées sont :

$$f_\alpha^x(\cdots) = \frac{1}{M_\alpha^{\star x}} \cdot \Bigg[ \quad \mu(\alpha) \cdot \Big( \sum_{k \in \Lambda(\alpha)} \varphi_k^x \Big)/|\Lambda(\alpha)|$$

$$- \sum_{k \in \Lambda(\alpha)} \mu(k) \cdot \Big\{ \Big( \sum_{\substack{\beta \in \Lambda(k) \\ \beta \neq \alpha}} \varphi_\beta^x \Big)/|\Lambda(k)|^2 - \varphi_k^x/|\Lambda(k)| \Big\}$$

$$- \sum_i \{ \Gamma_i^{x\alpha} - Q_i^{x\alpha} \} \quad \Bigg]$$

$$f_\alpha^y(\cdots) = \frac{1}{M_\alpha^{\star y}} \cdot \Bigg[ \quad \mu(\alpha) \cdot \Big( \sum_{k \in \Lambda(\alpha)} \varphi_k^y \Big)/|\Lambda(\alpha)|$$

$$- \sum_{k \in \Lambda(\alpha)} \mu(k) \cdot \Big\{ \Big( \sum_{\substack{\beta \in \Lambda(k) \\ \beta \neq \alpha}} \varphi_\beta^y \Big)/|\Lambda(k)|^2 - \varphi_k^y/|\Lambda(k)| \Big\}$$

$$- \sum_i \{ \Gamma_i^{y\alpha} - Q_i^{y\alpha} \} \quad \Bigg]$$

$$f_\alpha^z(\cdots) = \frac{1}{M_\alpha^{\star z}} \cdot \Bigg[ \quad \mu(\alpha) \cdot \Big( \sum_{k \in \Lambda(\alpha)} \varphi_k^z \Big)/|\Lambda(\alpha)|$$

$$- \sum_{k \in \Lambda(\alpha)} \mu(k) \cdot \Big\{ \Big( \sum_{\substack{\beta \in \Lambda(k) \\ \beta \neq \alpha}} \varphi_\beta^z \Big)/|\Lambda(k)|^2 - \varphi_k^z/|\Lambda(k)| \Big\}$$

$$- \sum_i \{ \Gamma_i^{z\alpha} - Q_i^{z\alpha} \} \quad \Bigg]$$

$$\text{avec :} \quad \begin{cases} M_\alpha^{\star x} = M_\alpha + \sum_i \gamma_i^{x\alpha} \\[2mm] M_\alpha^{\star y} = M_\alpha + \sum_i \gamma_i^{y\alpha} \\[2mm] M_\alpha^{\star z} = M_\alpha + \sum_i \gamma_i^{z\alpha} \\[2mm] M_\alpha = \mu(\alpha) + \sum_{k \in \Lambda(\alpha)} \{\mu(k)/|\Lambda(k)|^2\} \end{cases}$$

Le procédé de calcul des valeurs $f_\alpha^x$, $f_\alpha^y$ et $f_\alpha^z$ prises par ces fonctions au noeud $\vec{\varphi}_\alpha$ est alors quasiment identique à celui décrit pour les pondérateurs harmoniques :

0) Allouer les mémoires de travail suivantes :

$$f_\alpha^x, f_\alpha^y, f_\alpha^z$$

$$\sigma_\varphi^x, \sigma_\varphi^y, \sigma_\varphi^z$$

$$s_\varphi^x, s_\varphi^y, s_\varphi^z$$

$$M_\alpha^{*x}, M_\alpha^{*y}, M_\alpha^{*z}, M_\alpha$$

$$\sigma_\mu, n_k^2$$

1) Effectuer les initialisations suivantes (l'ordre n'a aucune importance) :

$$f_\alpha^x = f_\alpha^y = f_\alpha^z = 0$$

$$\sigma_\varphi^x = \sigma_\varphi^y = \sigma_\varphi^z = 0$$

2) Déterminer le nombre $n_\alpha$, de satellites de $A(\alpha)$. Ce nombre peut être obtenu à partir du champ **nb_sat** de $A(\alpha)$

3) Soit $\Lambda(\alpha)$ l'ensemble des satellites de l'atome $A(\alpha)$ auxquels on accède par le champ **sat** de cet atome. Pour chaque satellite $k \in \Lambda(\alpha)$, répéter les opérations du bloc suivant :

{

3.1) Déterminer le nombre $n_k$ de satellites de $A(k)$. Ce nombre peut être obtenu à partir du champ **nb_sat** de $A(k)$. Effectuer ensuite l'opération suivante :

$$n_k^2 \leftarrow n_k \times n_k$$

3.2) Déterminer les coordonnées $(\varphi_k^x, \varphi_k^y, \varphi_k^z)$ du noeud $\vec{\varphi}_k$ de l'atome $A(k)$. Ces coordonnées peuvent être obtenues à partir du champ **noeud** de $A(k)$.

3.3) A partir des informations contenues dans $A(k)$, calculer ou déterminer la valeur du pondérateur $\mu(k)$ (voir section 1.1.4 et annexe).

3.4) Effectuer les opérations suivantes (l'ordre n'a aucune importance).

$$\sigma_\mu \leftarrow \sigma_\mu + \mu(k)/n_k^2$$
$$\sigma_\varphi^x \leftarrow \sigma_\varphi^x + \varphi_k^x$$
$$\sigma_\varphi^y \leftarrow \sigma_\varphi^y + \varphi_k^y$$
$$\sigma_\varphi^z \leftarrow \sigma_\varphi^z + \varphi_k^z$$
$$s_\varphi^x = s_\varphi^y = s_\varphi^z = 0$$

3.5) Soit $\Lambda(k)$ l'ensemble des satellites de l'atome $A(k)$ auquel on accède par le champ **sat** de cet atome. Pour chaque satellite $\beta \in \Lambda(k)$, $\beta \neq \alpha$ effectuer les opérations du bloc suivant :

{

3.5.2) Déterminer les coordonnées actuelles $(\varphi_\beta^x, \varphi_\beta^y, \varphi_\beta^z)$ du noyau $\vec{\varphi}_\beta$ de l'atome $A(\beta)$. Ces coordonnées peuvent être obtenues à partir du champ **noeud** de $A(\beta)$.

3.5.3) Effectuer les 3 opérations suivantes (l'ordre n'a aucune importance) :

$$s_\varphi^x \leftarrow s_\varphi^x + \varphi_\beta^x$$
$$s_\varphi^y \leftarrow s_\varphi^y + \varphi_\beta^y$$
$$s_\varphi^z \leftarrow s_\varphi^z + \varphi_\beta^z$$

}

3.6) Effectuer les 3 opérations suivantes (l'ordre n'a aucune importance) :

$$\left|\begin{array}{l} f_\alpha^x \leftarrow f_\alpha^x + \mu(k) \times (s_\varphi^x/n_k^2 - \varphi_k^x/n_k) \\ f_\alpha^y \leftarrow f_\alpha^y + \mu(k) \times (s_\varphi^y/n_k^2 - \varphi_k^y/n_k) \\ f_\alpha^z \leftarrow f_\alpha^z + \mu(k) \times (s_\varphi^z/n_k^2 - \varphi_k^z/n_k) \end{array}\right. \qquad \vec{\varphi}_\beta$$

}

4) Effectuer les opérations suivantes :

$$\left|\begin{array}{lcl} M_\alpha & \leftarrow & \mu(\alpha) + \sigma_\mu \\ M_\alpha^{*x} & \leftarrow & M_\alpha \\ M_\alpha^{*y} & \leftarrow & M_\alpha \\ M_\alpha^{*z} & \leftarrow & M_\alpha \end{array}\right.$$

5) Soit $C(\alpha)$ l'ensemble des contraintes attachées à l'atome $A(\alpha)$ auquel on accède par le champ **const** de cet atome. Pour chaque contrainte $c \in C(\alpha)$ répéter les opérations du bloc suivant :

{

5.1) Déterminer le type de la contrainte $c$

5.2) En fonction du type de la contrainte $c$, effectuer les deux opérations suivantes :

5.2.1) Extraire les informations spécifiques de la contrainte $c$

5.2.2) En fonction des informations extraites en (5.2.1), calculer les valeurs des expressions suivantes (voir sections 2.5 et 2.7 de l'annexe) :

$$\left|\begin{array}{l} \Gamma_i^{x\alpha}, \Gamma_i^{y\alpha}, \Gamma_i^{z\alpha} \\ Q_i^{x\alpha}, Q_i^{y\alpha}, Q_i^{z\alpha} \\ \gamma_i^{x\alpha}, \gamma_i^{y\alpha}, \gamma_i^{z\alpha} \end{array}\right.$$

5.3) Effectuer les opérations suivantes (l'ordre n'a aucune importance) :

$$\left|\begin{array}{l} M_\alpha^{*x} \leftarrow M_\alpha^{*x} + \gamma_i^{x\alpha} \\ M_\alpha^{*y} \leftarrow M_\alpha^{*y} + \gamma_i^{y\alpha} \\ M_\alpha^{*z} \leftarrow M_\alpha^{*z} + \gamma_i^{z\alpha} \\ f_\alpha^x \leftarrow f_\alpha^x + (\Gamma_i^{x\alpha} - Q_i^{x\alpha}) \\ f_\alpha^y \leftarrow f_\alpha^y + (\Gamma_i^{y\alpha} - Q_i^{y\alpha}) \\ f_\alpha^z \leftarrow f_\alpha^z + (\Gamma_i^{z\alpha} - Q_i^{z\alpha}) \end{array}\right.$$

}

6) Effectuer les opérations suivantes (l'ordre n'a aucune importance) :

$$\left|\begin{array}{l} f_\alpha^x \leftarrow (\mu(\alpha) \times \sigma_\varphi^x/n_\alpha - f_\alpha^x)/M_\alpha^{*x} \\ f_\alpha^y \leftarrow (\mu(\alpha) \times \sigma_\varphi^y/n_\alpha - f_\alpha^y)/M_\alpha^{*y} \\ f_\alpha^z \leftarrow (\mu(\alpha) \times \sigma_\varphi^z/n_\alpha - f_\alpha^z)/M_\alpha^{*z} \end{array}\right.$$

Les paramètres numériques stockés dans le champ info des atomes peuvent également être interpolé par la méthode DSI (voir section 2.1.2 et 2.2 de l'annexe). Dans ce cas, la structure du procédé d'interpolation est quasiment identique à celle du procédé d'ajustement de la surface presenté ci-dessus: le seul changement consiste à ne conserver qu'une des trois fonctions d'ajustement normalement dédiées aux trois coordonnées des noeuds et à calculer celle-ci en fonction du paramètre interpolé.

La présente invention trouve application tout particulièrement dans les domaines suivants :

- En géologie et en géophysique, elle permet de créer un modèle de surface, par exemple de construire une maquette solide en trois dimensions, sur laquelle on peut effectuer une simulation géophysique ou une simulation d'exploitation de richesses souterraines; elle permet également d'obtenir un ensemble de données propres à initialiser un simulateur numérique ou analogique de phénomènes géophysiques ou d'exploitation de richesses; en outre, elle permet, à l'aide d'une station de travail graphique, d'afficher sur un écran ou d'imprimer sur un support des images permettant de contrôler les simulations ci-dessus ;
- en biologie ou en médecine, l'invention permet de construire des modèles en trois dimensions d'organes et/ou de prothèses, ou encore de simuler les effets d'opérations en chirurgie plastique: de même, des images en deux dimensions affichées ou imprimées permettent ici encore de contrôler les opérations ci-dessus. En particulier, la surface modélisée peut être représentative des variations d'une propriété d'un corps à trois dimensions au voisinage d'un plan traversant ledit corps, ladite propriété étant représentée par la dimension restante.

Mais il est clair que l'invention s'applique plus généralement à la modélisation de surfaces de type quelconque, et notamment de toute surface naturelle ou industrielle. Elle peut trouver un intérêt tout particulier dans le cadre de la conception assistée par ordinateur (CAO).

## ANNEXE

## 2. NOUVELLE VERSION DE LA METHODE DSI ADAPTEE A LA MODELISATION INTERACTIVE DES SURFACES COMPLEXES.

*Dans les domaines de la Géologie et de la Biologie, il est fréquent d'avoir à modéliser des surfaces complexes correspondant par exemple à des interfaces entre zones de nature ou de propriétés différentes. Les techniques classiques de modélisation basées sur les interpolations de Bézier ou sur les fonctions Splines (voir ref. [9]) sont trés mal adaptées à la prise en compte de ces données hétérogènes. Nous proposons ici une approche différente basée sur la méthode DSI (Discrete Smooth Interpolation - voir ref. [8]). Dans cette approche, les surfaces sont modélisées avec des facettes triangulaires irrégulières dont les sommets doivent être déterminés afin de tenir compte du plus grand nombre de données hétérogènes possible.*

### 2.1 Introduction :

### Présentation des problèmes étudiés

### 2.1.1 Surface $S$ définitions associées

Comme le montre la Figure 1, soit $S$ une surface composée de facettes triangulaires adjacentes plongée dans l'espace euclidien 3D ($O\,|\,x, y, z$). Cette surface n'est pas forcément connexe ni fermée. Dans ce qui suit, nous utiliserons les notations suivantes :

$$\begin{cases} G & = & G(S) & = & \text{ensemble des cotés des triangles} \\ \Omega & = & \Omega(S) & = & \text{ensemble des sommets des triangles} \\ N & = & |\Omega| & = & \text{nombre de points de } \Omega \end{cases}$$

$G$ est un graphe dont les noeuds sont les points de $\Omega$ et nous identifierons cet ensemble avec l'ensemble des indices $\{1,...,N\}$ utilisés pour les numéroter. Le voisinage $N(k)$ d'un noeud $k \in \Omega$ est défini par :

$$\left| \begin{array}{l} N(k) & = & \text{sous ensemble de } \Omega \text{ constitué par les noeuds de} \\ & & \Omega \text{ qui peuvent être atteints à partir du noeud } k \\ & & \text{en } s(k) \text{ pas au plus où } s(k) \text{ est un nombre positif} \\ & & \text{donné.} \end{array} \right.$$

Dans ce qui suit, nous supposerons que ces voisinages $N(k)$ possèdent la propriété de **symétrie** suivante :

$$\alpha \in N(\beta) \Leftrightarrow \beta \in N(\alpha)$$

Les voisinages ainsi définis engendrent une topologie sur $G$ qui peut être considérée comme une approximation de la topologie de $S$ elle même.

### 2.1.2 Problème 1 : Estimation d'une fonction $\varphi$ définie sur $\Omega$

Supposons que la position de chaque noeud $k \in \Omega$ est connue et soit $\varphi(k)$ une fonction définie pour chaque noeud $k \in \Omega$ et connue seulement sur un sous-ensemble de $\Omega$ :

$$\left|\begin{array}{lllllll} L & = & \text{ensemble des noeuds} & l \in \Omega & \text{où} & \varphi(l) & = & \varphi_l \text{ est connu} \\ I & = & \text{ensemble des noeuds} & i \in \Omega & \text{où} & \varphi(i) & = & \varphi_i \text{ est inconnu} \\ & = & \Omega - L \end{array}\right.$$

Généralement, $L$ est différent de $\Omega$ et il y a une infinité de fonctions $\varphi(k)$ définies sur $\Omega$ et interpolant les valeurs $\{\varphi(l)=\varphi_l : l \in L\}$. Notre objectif est de selectionner parmi toutes ces fonctions celle qui minimise un critère donné $R^*(\varphi)$ mesurant :
- la rugosité globale de la fonction $\varphi(\cdot)$
- l'écart entre la fonction $\varphi(\cdot)$ et un certain nombre de contraintes et données qu'elle doit satisfaire

### 2.1.3 Problème 2 : Ajustement de la surface $S$

Dans ce cas, seuls les points d'un sous ensemble $L$ de noeuds $k \in \Omega$ ont une position connue $\vec{\varphi}(k) = \vec{\varphi}_k$ :

$$\left|\begin{array}{lllllll} L & = & \text{ensemble de noeuds} & l \in \Omega & \text{où} & \vec{\varphi}(l) & = & \vec{\varphi}_l \text{ est connu} \\ I & = & \text{ensemble des noeuds} & i \in \Omega & \text{où} & \vec{\varphi}(i) & = & \vec{\varphi}_i \text{ est inconnu} \\ & = & \Omega - L \end{array}\right.$$

Comme dans le cas du premier problème, $L$ est généralement différent de $\Omega$ et il y a une infinité de surfaces $S$ interpolant les points $\{\vec{\varphi}(l) = \varphi_l : l \in L\}$. Notre objectif est de sélectionner parmi toutes ces surfaces, celle qui minimise un critère $R^*(\vec{\varphi})$ donné mesurant :
- la rugosité globale de la surface $S$
- l'écart entre la surface $S$ et un certain nombre de contraintes et données qu'elle doit satisfaire

Compte tenu de l'indépendance des composantes $(\varphi^x(k), \varphi^y(k), \varphi^z(k))$ de chaque vecteur $\vec{\varphi}(k)$, il est facile d'utiliser la solution du premier problème pour résoudre le second en posant simplement :

$$R^*(\vec{\varphi}) = R^*(\varphi^x) + R^*(\varphi^y) + R^*(\varphi^z)$$

Dans le cas très particulier ou l'ensemble $\Omega$ correspond aux noeuds d'une grille rectangulaire posée sur la surface, on peut songer à utiliser une méthode d'interpolation du type spline, Briggs ou Bézier (Voir ref. [9], [1] et [3]); malheureusement ces méthodes sont inapplicables dans le cas des grilles irrégulières et ne permettent pas de tenir compte des contraintes présentées aux paragraphes 2.2.3, 2.5 et 2.7.2.

### 2.2 Interpolation d'une fonction $\varphi(k)$ définie sur $\Omega$

Dans cette section, nous introduisons brièvement la méthode DSI (Discrete Smooth Interpolation) presentée dans l'article [8].

### 2.2.1 Notations

Dans ce qui suit, nous noterons $\varphi$ une matrice colonne de taille $N$ telle que :

$$\left|\begin{array}{rcl} \varphi & = & [\varphi_1, \ldots, \varphi_N]^t \\ \varphi_k & = & \varphi(k) \quad \forall k \in \Omega \end{array}\right.$$

Notre problème ne dépend pas de la méthode de numérotation des noeuds de la grille. Afin de simplifier les notations, nous supposerons que les éléments de la matrice $\varphi$ ont été éventuellement permutés de telle façon que $\varphi$ puisse être partagée en deux sous-matrices $\varphi_I$ et $\varphi_L$ telles que :

$$\varphi = \left[\begin{array}{c} \varphi_I \\ \varphi_L \end{array}\right] \Longleftrightarrow \left\{\begin{array}{ll} \varphi_I = \left[\begin{array}{c} \varphi_{i_1} \\ \vdots \\ \varphi_{i_n} \end{array}\right] & \text{avec} \quad i_\alpha \in I \quad \forall \, \alpha \\ \varphi_L = \left[\begin{array}{c} \varphi_{l_1} \\ \vdots \\ \varphi_{l_m} \end{array}\right] & \text{avec} \quad l_\beta \in L \quad \forall \, \beta \end{array}\right.$$

De plus, nous noterons $\| \cdot \|_D$ une semi-norme associée à une matrice carrée semi-définie positive $[D]$ de taille $(N \times N)$ de telle façon que, pour toute matrice colonne $X$ de taille $N$ on ait :

$$\|X\|_D^2 = X^t \cdot [D] \cdot X$$

## 2.2.2 Définition d'un critère de rugosité globale $R(\varphi)$

Soit $R(\varphi \,|\, k)$ le critère de rugosité locale défini par la formule suivante où les $\{ v^\alpha (k)\}$ sont des coefficients de pondération positifs, négatifs ou nuls **donnés** :

$$R(\varphi|k) = | \sum_{\alpha \in N(k)} v^\alpha(k) \cdot \varphi_\alpha |^2$$

$R(\varphi \,|\, k)$ peut être utilisé pour construire un critère de rugosité globale $R(\varphi)$ défini de la façon suivante où $\mu(k)$ est une fonction de pondération **donnée** définie sur $\Omega$ :

$$R(\varphi) = \sum_k \mu(k) \cdot R(\varphi|k)$$

$R(\varphi)$ est complètement défini par la donnée des coefficients $\{v^\alpha {}_{|}(k)\}$ et $\mu(k)$ et il est facile de vérifier (voir ref. [8]) qu'il existe toujours une matrice $(N \times N)$ symétrique semi-définie positive $[W]$ telle que :

$$R(\varphi \,|\, k) = \varphi^t \cdot [W(k)] \cdot \varphi$$

## 2.2.3 Prise en compte de contraintes linéaires au sens des moindres carrés

Considérons une matrice carrée $[A_i]$ de taille $(N \times N)$ et une matrice colonne $B_i$ de taille $N$ définissant la contrainte linéaire suivante :

$$[A_i] \cdot \varphi \simeq B_i$$

Etant donnée une matrice semi-définie positive $[D_i]$, le symbole '$\simeq$" figurant dans la formule ci-dessus a la signification suivante :

$$\| [A_i] \cdot \varphi - B_i \|_{D_i}^2 \text{ aussi petit que possible}$$

S'il existe plusieurs conditions de ce type, nous proposons de mesurer le degré de violation de ces contraintes par le critère $\rho(\varphi)$ défini comme suit :

$$\rho(\varphi) \;=\; \sum_i \|\,[A_i]\cdot\varphi - B_i\,\|^2_{D_i}$$

### 2.2.4 Solution du problème

Parmi toutes les fonctions $\varphi(k)$ définies sur $\Omega$ et interpolant les données $\{\varphi(l) = \varphi_l : l{\in}L\}$, nous en choisirons une qui minimise le critère suivant :

$$R^*(\varphi) \;=\; R(\varphi) \;+\; \rho(\varphi)$$

En développant $R^*(\varphi)$, on obtient :

$$
R^\star(\varphi) \;=\; \varphi^t\cdot[W^\star]\cdot\varphi \;-\; 2\cdot\varphi^t\cdot[Q] \;+\; C
$$

$$
\text{avec}\;\;
\begin{cases}
[W^\star] &= \displaystyle\sum_i [A_i]^t\cdot[D_i]\cdot[A_i] \;+\; [W]\\[2ex]
Q &= \displaystyle\sum_i [A_i]^t\cdot[D_i]\cdot B_i\\[2ex]
C &= \displaystyle\sum_i \|\,B_i\,\|^2_{D_i}
\end{cases}
$$

La partition de la matrice $\varphi$ induit une partition similaire des matrices $[W^*]$ et $Q$ utilisées pour définir $R^*(\varphi)$ :

$$
\varphi \;=\; \begin{bmatrix}\varphi_I\\ \varphi_L\end{bmatrix}
\;\Longrightarrow\;
[W^*] \;=\; \begin{bmatrix}W^\star_{II} & W^\star_{IL}\\ W^\star_{LI} & W^\star_{LL}\end{bmatrix}
\;;\;
Q \;=\; \begin{bmatrix}Q_I\\ Q_L\end{bmatrix}\;.
$$

La condition $\partial R^*(\varphi)/\partial\varphi_I = [0]$ implique l'équation suivante appelée "équation DSI" et caractérisant toutes les fonctions $\{\varphi(k) : k \in \Omega\}$ solution de notre problème :

$$
(\text{DSI}) : \;
\begin{cases}
[W^\star_{II}]\cdot\varphi_I &= \psi_I\\[2ex]
\text{avec} : \psi_I &= -[W^\star_{IL}]\cdot\varphi_L + Q_I
\end{cases}
$$

### 2.3 Unicité de la solution de l'équation DSI

**Définition**

L'ensemble $L$ des noeuds où $\varphi$ est connu sera dit **consistant** relativement à $R^*(\varphi)$ si chaque composante connexe du graphe $G$ contient au moins un noeud appartenant à $L$.

**théorème**

Si le critère de rugosité globale $R(\varphi)$ est tel que :

$$\left|\begin{array}{llll} 1) & L & & \text{est consistant relativement à } R(\varphi) \\ 2) & \mu(k) & > 0 & \forall\, k \\ 3) & v^\alpha(k) & \geq 0 & \forall\, \{\alpha \neq k, \alpha \in N(k)\} \\ 4) & v^k(k) & \leq - \displaystyle\sum_{\substack{\alpha \in N(k) \\ \alpha \neq k}} v^\alpha(k) \neq 0 \end{array}\right.$$

alors, l'équation DSI basée sur $R(\varphi)$ admet une solution unique.

**Démonstration**

Le théorème ci-dessus ne présente qu'un intérêt théorique et n'a aucune incidence sur le procédé décrit dans le présent brevet, c'est pourquoi nous ne donnerons pas ici sa démonstration.

**2.4 Forme locale de l'équation DSI**

Dans ce qui suit, plutôt que de résoudre directement l'équation DSI, nous proposons une approche itérative originale permettant d'éviter le calcul et le stockage de [$W^*$]. Cette approche itérative est précisément celle qui est utilisée dans le présent brevet.

Afin de simplifier les notations, nous supposerons dans ce qui suit que les matrices semi-définies positives [$D_i$] intervenant dans l'équation DSI sont **diagonales**. De plus, lorsqu'il y a des contraintes linéaires du type [$A_i$]·$\varphi \simeq B_i$, nous utiliserons les notations suivantes :

$$\left[\begin{array}{lll} A_i^{kh} & = & \text{élément de } [A_i] \text{ situé sur la } \left\{\begin{array}{ll} \text{ligne} & k \\ \text{colonne} & h \end{array}\right. \\ Q_i^\alpha & = & \alpha^{th} \text{ élément de la matrice colonne } ([A_i]^t [D_i] B_i) \end{array}\right.$$

**2.4.1 Calcul de $\partial R^*(\varphi)/\partial\varphi_\alpha$**

Compte tenu de la définition de $R(\varphi\,|\,k)$, on peut écrire l'implication suivante :

$$\begin{aligned} R(\varphi|k) &= \sum_{\beta \in N(k)} \sum_{\gamma \in N(k)} v^\beta(k)\, v^\gamma(k) \cdot \varphi_\beta \varphi_\gamma \\ &= \sum_{\beta \in N(k)} (v^\beta(k))^2 \cdot \varphi_\beta^2 + \sum_{\gamma \in N(k)} \sum_{\substack{\beta \in N(k) \\ \beta \neq \gamma}} v^\beta(k)\, v^\gamma(k) \cdot \varphi_\beta \varphi_\gamma \end{aligned}$$

$$\implies \frac{\partial R(\varphi|k)}{\partial \varphi_\alpha} = \left\{\begin{array}{ll} 2 \cdot (v^\alpha(k))^2 \cdot \varphi_\alpha + 2 \cdot v^\alpha(k) \cdot \displaystyle\sum_{\substack{\beta \in N(k) \\ \beta \neq \alpha}} v^\beta(k) \cdot \varphi_\beta & \text{si } \alpha \in N(k) \\ \\ 0 & \text{sinon} \end{array}\right.$$

On déduit de cette relation que :

$$\frac{1}{2} \cdot \frac{\partial R(\varphi)}{\partial \varphi_\alpha} = \frac{1}{2} \sum_k \mu(k) \cdot \frac{\partial R(\varphi|k)}{\partial \varphi_\alpha} = \frac{1}{2} \sum_{k \in N(\alpha)} \mu(k) \cdot \frac{\partial R(\varphi|k)}{\partial \varphi_\alpha}$$

$$= \varphi_\alpha \cdot \sum_{k \in N(\alpha)} \mu(k)\, (v^\alpha(k))^2 + \sum_{k \in N(\alpha)} \left\{ \mu(k)\, v^\alpha(k) \cdot \sum_{\substack{\beta \in N(k) \\ \beta \neq \alpha}} v^\beta(k) \cdot \varphi_\beta \right\}$$

Compte tenu de la structure diagonale de $[D_i]$, il est facile de vérifier que le $\alpha^{\text{ème}}$ élément de $\partial \parallel [A_i] \cdot \varphi - B_i \parallel^2_{D_i} / \partial \varphi_\alpha$ est tel que :

$$\frac{1}{2} \cdot \frac{\partial \parallel [A_i] \cdot \varphi - B_i \parallel^2_{D_i}}{\partial \varphi_\alpha} = \sum_k \left\{ A_i^{k\alpha} D_i^{kk} \cdot \sum_\beta A_i^{k\beta} \varphi_\beta \right\} - Q_i^\alpha$$

$$= \varphi_\alpha \cdot \sum_k D_i^{kk}\, (A_i^{k\alpha})^2 + \sum_k \left\{ A_i^{k\alpha} D_i^{kk} \cdot \sum_{\beta \neq \alpha} A_i^{k\beta} \varphi_\beta \right\} - Q_i^\alpha$$

Compte tenu de la définition de $R^*(\varphi)$, on en déduit que :

$$\frac{1}{2} \cdot \frac{R^*(\varphi)}{\partial \varphi_\alpha} = \varphi_\alpha \cdot \left\{ \sum_{k \in N(\alpha)} \mu(k)\, (v^\alpha(k))^2 + \sum_i \sum_k D_i^{kk}\, (A_i^{k\alpha})^2 \right\}$$
$$+ \sum_{k \in N(\alpha)} \left\{ \mu(k)\, v^\alpha(k) \cdot \sum_{\substack{\beta \in N(k) \\ \beta \neq \alpha}} v^\beta(k) \cdot \varphi_\beta \right\}$$
$$+ \sum_i \sum_k \left\{ A_i^{k\alpha} D_i^{kk} \cdot \sum_{\beta \neq \alpha} A_i^{k\beta} \varphi_\beta \right\}$$
$$- \sum_i Q_i^\alpha$$

La solution $\varphi$, est telle que $\partial R^*(\varphi)/\partial \varphi_\alpha = 0$, donc la $\alpha^{\text{ème}}$ composante $\varphi_\alpha$ de $\varphi$ doit satisfaire l'équation DSI($\alpha$) suivante :

$$\text{DSI}(\alpha) : \left[ \begin{array}{l} \varphi_\alpha = -\dfrac{1}{M_\alpha^\star} \cdot \left\{ \displaystyle\sum_{k \in N(\alpha)} \{\mu(k)\, v^\alpha(k) \cdot \displaystyle\sum_{\substack{\beta \in N(k) \\ \beta \neq \alpha}} v^\beta(k) \cdot \varphi_\beta\} \right. \\ \qquad\qquad\qquad \left. + \displaystyle\sum_i \{\Gamma_i^\alpha - Q_i^\alpha\} \right\} \\[2em] \text{avec :} \left\{ \begin{array}{l} M_\alpha^\star = M_\alpha + \displaystyle\sum_i \gamma_i^\alpha \\ M_\alpha = \displaystyle\sum_{k \in N(\alpha)} \mu(k)\, (v^\alpha(k))^2 \\ \gamma_i^\alpha = \displaystyle\sum_k D_i^{kk}\, (A_i^{k\alpha})^2 \\ \Gamma_i^\alpha = \displaystyle\sum_k \left\{ A_i^{k\alpha} D_i^{kk} \cdot \displaystyle\sum_{\beta \neq \alpha} A_i^{k\beta} \varphi_\beta \right\} \\ Q_i^\alpha = \displaystyle\sum_k A_i^{k\alpha} D_i^{kk} B_i^k \end{array} \right. \end{array} \right.$$

Nous proposons d'appeler cette équation *forme locale de l'équation DSI* au noeud $\alpha$.

### 2.4.2 Proposition pour un algorithme itératif

La forme locale de l'équation DSI présentée dans la section précédente suggère un algorithme évident pour estimer la solution $\varphi$. Par exemple, à l'itération ($n$+1), la $\alpha^{\text{ème}}$ composante $\varphi_\alpha^{(n+1)}$ de la solution $\varphi^{(n+1)}$ doit satisfaire l'équation DSI($\alpha$) ce qui permet de proposer le procédé itératif suivant :

**soit** $I$ l'ensemble des indices de noeuds où $\varphi_\alpha$ est inconnu

**soit** $\varphi$ une solution approchée initiale

**tant_que**( il est nécessaire de faire de nouvelles itérations )
{

    **pour_tout**( $\alpha \in I$ )
    {

$$M_\alpha^\star = \{\cdots\}$$
$$\varphi_\alpha = -\frac{1}{M_\alpha^\star} \cdot \{\cdots\}$$

    } .
}

Comme on peut le voir, cet algorithme très simple n'utilise pas explicitement la matrice [$W^\star$] intervenant dans l'équation DSI mais il requiert de recalculer un grand nombre de fois des produits du type { $v^\alpha$ ($k$)· $v^\beta$ ($k$)} qui sont précisément ceux utilisés pour construire [$W$].

En fait, si la solution approchée initiale est proche de la solution exacte, alors il n'est pas nécessaire d'effectuer beaucoup d'itérations et le surcoût de calcul devient négligeable. Par exemple, cette situation se présente dans un contexte interactif où la solution initiale $\varphi$ est prise égale à la solution avant que quelques modifications locales soient effectuées par l'utilisateur. Dans ce cas, malgré le léger surcoût engendré, la forme locale de l'équation DSI est préférable car elle est bien plus facile à mettre en oeuvre que la forme globale.

Afin de montrer que l'algorithme proposé converge, on peut remarquer que $R^\star(\varphi)$ peut être exprimé sous forme d'une fonction de $\varphi_\alpha$ ayant la forme suivante :

$$R^\star(\varphi) = A \cdot \varphi_\alpha^2 + B \cdot \varphi_\alpha + C$$

Les coefficients $A$, $B$ et $C$ sont indépendants de $\varphi_\alpha$ et on a :

$$\begin{cases} A & = & \displaystyle\sum_{k \in N(\alpha)} \mu(k) \, (v^\alpha(k))^2 + \sum_i \gamma_i^\alpha \\[3em] B & = & 2 \cdot \left\{ \displaystyle\sum_{k \in N(\alpha)} \{ \mu(k) \, v^\alpha(k) \cdot \sum_{\substack{\beta \in N(k) \\ \beta \neq \alpha}} v^\beta(k) \cdot \varphi_\beta \} + \sum_i \{ \Gamma_i^\alpha - Q_i^\alpha \} \right\} \end{cases}$$

Le minimum du critère global $R^*(\varphi)$ est atteint pour la valeur $\varphi_\alpha = -B/(2A)$ qui correspond précisément à la valeur fournie par l'équation DSI($\alpha$). Ceci nous permet de conclure que notre algorithme converge car, à chaque itération du procéssus itératif, la valeur de la fonction positive ou nulle $R^*(\varphi)$ décroit :

## 2.5 Prise en compte de données imprécises

Dans ce qui suit, nous introduisons deux types de contraintes linéaires qui sont d'une extrème importance en modélisation géométrique ainsi qu'on le verra au paragraphe 2.7.6. L'objectif de cette section est de prendre en compte des données imprécises concernant les valeurs inconnues $\{\varphi\lambda : \lambda \in I\}$. De telles données sont supposées associées à des *coefficients de certitude* $\overline{\omega}^2 \in \mathbf{R}^+$ qui sont en fait des coefficients de pondération positifs.

### 2.5.1 Cas d'une donnée ponctuelle imprécise

Soit $\lambda \in I$ un indice de noeud pour lequel la valeur inconnue $\varphi_\lambda$ est supposée proche d'une donnée incertaine $\tilde{\varphi}_\lambda$ avec un coefficient de certitude donné egal à $\overline{\omega}_\lambda^2$ :

$$\varphi_\lambda \simeq \tilde{\varphi}_\lambda$$

ou $\simeq$ correspond à la condition suivante : $\varphi_\lambda$ :

$$\overline{\omega}_\lambda^2 \cdot | \varphi_\lambda^* - \tilde{\varphi}_\lambda |^2 \text{ minimum}$$

Une telle condition est aisément prise en compte par la méthode DSI sous forme d'une $i^{\text{ème}}$ contrainte avec :

$$A_i^{kh} = \begin{cases} +1 & \text{si } k = h = \lambda \\ 0 & \text{sinon} \end{cases} \qquad B_i^k = \begin{cases} \tilde{\varphi}_\lambda & \text{si } k = \lambda \\ 0 & \text{sinon} \end{cases}$$

$$\text{et} \quad D_i^{kh} = \begin{cases} \varpi_\lambda^2 & \text{si } k = h = \lambda \\ 0 & \text{sinon} \end{cases}$$

Les coefficients $\Gamma_i^\alpha$, $Q_i^\alpha$ et $\gamma_i^\alpha$ correspondants utilisés dans l'équation locale DSI($\alpha$) sont alors définis par :

$$\Gamma_i^\alpha = 0 \qquad Q_i^\alpha = \begin{cases} \varpi_\lambda^2 \cdot \tilde{\varphi}_\lambda & \text{si } \alpha = \lambda \\ 0 & \text{sinon} \end{cases}$$

$$\text{et} \quad \gamma_i^\alpha = \begin{cases} \varpi_\lambda^2 & \text{si } \alpha = \lambda \\ 0 & \text{sinon} \end{cases}$$

### 2.5.2 Cas d'une donnée différentielle imprécise

Soit $\lambda \in I$ un index de noeud pour lequel la valeur inconnue $\varphi\lambda$ est supposée liée à une autre valeur $\varphi_\mu$ correspondant à un autre index $\mu \neq \lambda$. Supposons que ce lien ait la forme suivante où $\Delta_{\lambda\mu}$ est une valeur donnée :

$$(\varphi_\mu - \varphi_\lambda) \simeq \Delta_{\lambda\mu}$$

Cette relation est supposée imprécise et est interprétée de la façon suivante où $\overline{\omega}^2_{\lambda\mu}$ est un coefficient de certitude :

$$\overline{\omega}^2_{\lambda\mu} \cdot |(\varphi^\star_\mu - \varphi^\star_\lambda) - \Delta_{\lambda\mu}|^2 \text{ minimum}$$

Une telle condition peut être facilement prise en compte par la méthode DSI en tant que $i^{\text{ème}}$ contrainte avec :

$$A^{kh}_i = \begin{cases} -1 & \text{si } k = h = \lambda \\ -1 & \text{si } k = h = \mu \\ +1 & \text{si } k = \lambda \text{ et } h = \mu \\ +1 & \text{si } k = \mu \text{ et } h = \lambda \\ 0 & \text{sinon} \end{cases} \qquad B^k_i = \begin{cases} + \ \Delta_{\lambda\mu} & \text{si } k = \lambda \\ - \ \Delta_{\lambda\mu} & \text{si } k = \mu \\ 0 & \text{sinon} \end{cases}$$

$$\text{et} \quad D^{kh}_i = \begin{cases} \frac{1}{2}\varpi^2_{\lambda\mu} & \text{si } k = h = \lambda \\ \frac{1}{2}\varpi^2_{\lambda\mu} & \text{si } k = h = \mu \\ 0 & \text{sinon} \end{cases}$$

Les coefficients $\Gamma^\alpha_i$, $Q^\alpha_i$ et $\gamma^\alpha_i$ correspondants utilisés dans l'équation locale DSI($\alpha$) sont alors définis par :

$$\Gamma^\alpha_i = \begin{cases} -\varpi^2_{\lambda\mu} \cdot \varphi^\star_\mu & \text{si } \alpha = \lambda \\ -\varpi^2_{\lambda\mu} \cdot \varphi^\star_\lambda & \text{si } \alpha = \mu \\ 0 & \text{sinon} \end{cases} \qquad Q^\alpha_i = \begin{cases} -\varpi^2_{\lambda\mu} \cdot \Delta_{\lambda\mu} & \text{si } \alpha = \lambda \\ +\varpi^2_{\lambda\mu} \cdot \Delta_{\lambda\mu} & \text{si } \alpha = \mu \\ 0 & \text{sinon} \end{cases}$$

$$\text{et} \quad \gamma^\alpha_i = \begin{cases} \varpi^2_{\lambda\mu} & \text{si } \alpha = \lambda \\ \varpi^2_{\lambda\mu} & \text{si } \alpha = \mu \\ 0 & \text{sinon} \end{cases}$$

### 2.5.3 Choix des coefficients de certitude

Considérons le terme $M^\star_\alpha$ de l'équation DSI($\alpha$) :

$$\begin{bmatrix} M^\star_\alpha = M_\alpha + \gamma^\alpha_1 + \gamma^\alpha_2 + \cdots \\ \text{avec} : M_\alpha = \sum_{k \in N(\alpha)} \mu(k) \, (v^\alpha(k))^2 \end{bmatrix}$$

Dans les deux exemples présentés ci-dessus, les termes $\gamma^\alpha_i$ sont soit égaux à zéro, soit correspondent au coefficient de certitude :

$$\gamma^\alpha_i = \overline{\omega}^2_i$$

Cette remarque nous suggère de choisir pour $\overline{\omega}_i^2$ un pourcentage donné $p_i$ de $M_\alpha$ :

Le terme $M_\alpha^*$ de l'équation DSI($\alpha$) est alors égal à :

$$M_\alpha^* = M_\alpha \cdot (1 + p_1(\alpha) + p_2(\alpha) + \cdots)$$

où $p_i(\alpha)$ est soit égal à un pourcentage donné $p_i$ ou égal à zéro.

## 2.6 Choix des coefficients de pondération

Le choix des coefficients de pondération $\{v^\alpha{}_i(k)\}$ et $\{\mu(k)\}$ est complètement libre excepté que les coefficients $\{\mu(k)\}$ doivent être positifs ou égaux à zéro. Dans ce qui suit nous donnons un exemple de choix de ces deux familles de coefficients.

### 2.6.1 Choix de coefficients $\{v^\alpha(k)\}$ : pondération harmonique

Soit $\Lambda(k)$ 'l"orbite" de $k$ définie de la façon suivante :

$$\Lambda(k) = N(k) - \{k\}$$

Soit $|\Lambda(k)|$ le nombre d'éléments de $\Lambda(k)$. Nous dirons que nous utilisons une pondération *harmonique* si les coefficients $\{ v^\alpha(k)\}$ sont choisis de la façon suivante :

$$v^\alpha(k) = \left\{ \begin{array}{lll} -|\Lambda(k)| & \text{si} & \alpha = k \\ 1 & \text{si} & \alpha \in \Lambda(k) \end{array} \right.$$

Les fonctions harmoniques ont la propriété caractéristique d'être égales en tout point à leur propre moyenne sur un cercle centré en ce point. Comme on peut le voir, $R(\varphi \,|\, k) = 0$ si $\varphi_k$ est égal à la moyenne des valeurs de $\varphi_\alpha$ entourant le noeud $k$, c'est pourquoi, par analogie avec les fonctions harmoniques, nous proposons d'appeller *harmoniques* les coefficients $\{ v^\alpha(k)\}$ presentés dans cette section.

Si nous développons l'équation DSI($\alpha$) avec ces coefficients, nous obtenons la formule suivante qui peut être facilement transcrite dans un langage de programmation :

$$\text{DSI}(\alpha) : \left[ \begin{array}{l} \varphi_\alpha = \dfrac{1}{M_\alpha^\star} \cdot \Big[ \quad \mu(\alpha) \cdot |\Lambda(\alpha)| \cdot \Big( \sum_{k \in \Lambda(\alpha)} \varphi_k \Big) \\[2ex] \qquad - \sum_{k \in \Lambda(\alpha)} \mu(k) \cdot \Big\{ \Big( \sum_{\substack{\beta \in \Lambda(k) \\ \beta \neq \alpha}} \varphi_\beta \Big) - |\Lambda(k)| \cdot \varphi_k \Big\} \\[2ex] \qquad - \sum_i \{ \Gamma_i^\alpha - Q_i^\alpha \} \quad \Big] \\[3ex] \text{avec :} \quad M_\alpha^\star = \mu(\alpha) \cdot |\Lambda(\alpha)|^2 + \sum_{k \in \Lambda(\alpha)} \mu(k) + \sum_i \gamma_i^\alpha \end{array} \right.$$

### 2.6.2 Choix des coefficients $\{\mu(k)\}$

Les coefficients $\{\mu(k)\}$ ont été introduits afin de moduler localement le lissage de l'interpolation. En l'absence de raison particulière, nous suggérons d'utiliser une pondération uniforme :

$$\mu(k) = 1 \ \forall \ k \in \Omega$$

Parmi toutes les pondérations non uniformes, la pondération suivante semble particulièrement intéressante s'il est necessaire d'avoir une fonction lisse au voisinage des données :

$$\mu(k) = \left\{ \begin{array}{lll} 1 & \text{si} & k \in I \\ m > 1 & \text{si} & k \in L \end{array} \right.$$

### 2.7 Application à la Modélisation Géométrique

Dans cette section, nous nous proposons de montrer comment utiliser la méthode DSI afin d'estimer la surface triangulée $S$ elle même pour laquelle on suppose disposer des informations partielles suivantes :

- la position exacte de quelques sommets de triangles,
- la position approchée de quelques sommets de triangles,
- des contraintes vectorielles précisant la forme de $S$,
- etc ...

Pour cela, on est amené à définir $\vec{\varphi}_\alpha$ comme le **vecteur courant** joignant l'origine de $\mathbf{R}^3$ au noeud **courant** de $\Omega$ et nous noterons $\varphi^x$, $\varphi^y$ et $\varphi^z$ ses composantes sur la base orthonormée $\{\vec{ox}, \vec{oy}, \vec{oz}\}$ de $\mathbf{R}^3$ :

$$\vec{\varphi} = (\varphi^x, \varphi^y, \varphi^z)$$

### 2.7.1 Définition d'un critère de rugosité globale $R(\vec{\varphi})$

Suivant notre habitude, l'ensemble $\Omega$ des noeuds de la grille est partagé en deux sous-ensembles $I$ et $L$

$$\left\{ \begin{array}{lll} \vec{\varphi}_i & \text{est inconnu} & \forall\, i \in I \\ \vec{\varphi}_l & \text{est connu} & \forall\, l \in L \end{array} \right.$$

Les noeuds $l \in L$ dont la position $\vec{\varphi}_l \in \mathbf{R}^3$ est connue seront appelés **noeud** de **contrôle** et l'on se propose de déterminer la position des points restants $i \in I$ de telle façon que le critère local $R(\vec{\varphi}\,|k)$ défini ci-dessous et basé sur un ensemble donné de pondérateurs $\{v^\alpha(k)\}$ soit aussi petit que possible :

$$R(\vec{\varphi}|k) = \left\| \sum_{\alpha \in N(k)} v^\alpha(k) \cdot \vec{\varphi}_\alpha \right\|^2$$

Ce critère constitue la forme vectorielle du critère DSI local. Le critère DSI global associé $(R\,\vec{\varphi})$ est alors défini de la façon suivante où les $\{\mu(k)\}$ sont des coefficients de pondération non négatifs donnés :

$$R(\vec{\varphi}) = \sum_k \mu(k) \cdot R(\vec{\varphi}|k)$$

Si l'on définit $R(\varphi^x)$, $R(\varphi^y)$ et $R(\varphi^z)$ ainsi qu'il a été fait dans les paragraphes précédents :

$$\begin{cases} R(\varphi^x) & = & \sum_k \mu(k) \cdot R(\varphi^x|k) & \text{avec}: R(\varphi^x|k) & = & |\sum_{\alpha \in N(k)} v^\alpha(k) \cdot \varphi_\alpha^x|^2 \\ R(\varphi^y) & = & \sum_k \mu(k) \cdot R(\varphi^y|k) & \text{avec}: R(\varphi^y|k) & = & |\sum_{\alpha \in N(k)} v^\alpha(k) \cdot \varphi_\alpha^y|^2 \\ R(\varphi^z) & = & \sum_k \mu(k) \cdot R(\varphi^z|k) & \text{avec}: R(\varphi^z|k) & = & |\sum_{\alpha \in N(k)} v^\alpha(k) \cdot \varphi_\alpha^z|^2 \end{cases}$$

alors, en utilisant le théorème de Pythagore, on vérifie facilement que l'on a :

$$R(\vec{\varphi}) = R(\varphi^x) + R(\varphi^y) + R(\varphi^z)$$

### 2.7.2 Prise en compte de contraintes linéaires au sens des moindres carrés

Considérons les matrices suivantes dimensionnées de telle façon que $N$ représente le nombre total de noeuds de l'ensemble $\Omega$ :

$$\forall \nu = x, y, z : \begin{cases} [D_{i_\nu}^\nu] & \text{est une matrice diagonale non négative de taille } N \times N \\ \\ [A_{i_\nu}^\nu] & \text{est une matrice carrée de taille } N \times N \\ \\ B_{i_\nu}^\nu & \text{est une matrice colonne de taille } N \end{cases}$$

Si nous voulons que les composantes $\varphi^x$, $\varphi^y$ et $\varphi^z$ satisfassent les contraintes suivantes

$$[A_{i_x}^x] \cdot \varphi^x \simeq B_{i_x}^x$$
$$[A_{i_y}^y] \cdot \varphi^y \simeq B_{i_y}^y$$
$$[A_{i_z}^z] \cdot \varphi^z \simeq B_{i_z}^z$$

alors on est amené à introduire le critère suivant

$$\rho(\vec{\varphi}) = \rho(\varphi^x) + \rho(\varphi^y) + \rho(\varphi^z)$$

$$\text{avec}: \begin{cases} \rho(\varphi^x) & = & \sum_{i_x} \| [A_{i_x}^x] \cdot \varphi^x - B_{i_x}^x \|_{D_{i_x}^x}^2 \\ \rho(\varphi^y) & = & \sum_{i_y} \| [A_{i_y}^y] \cdot \varphi^y - B_{i_y}^y \|_{D_{i_y}^y}^2 \\ \rho(\varphi^z) & = & \sum_{i_z} \| [A_{i_z}^z] \cdot \varphi^z - B_{i_z}^z \|_{D_{i_z}^z}^2 \end{cases}$$

### 2.7.3 Solution du problème

Parmi toutes les surfaces $S$ interpolant les données $\{ \vec{\varphi}(l) = \vec{\varphi}_l : l \in L \}$, nous nous proposons de sélectionner celle qui minimise le critère suivant :

$$R^\star(\vec{\varphi}) = R(\vec{\varphi}) + \rho(\vec{\varphi})$$

Il est facile de vérifier que l'on peut toujours décomposer cette formule de la façon suivante :

$$R^{\star}(\vec{\varphi}) = R^{\star}(\varphi^x) + R^{\star}(\varphi^y) + R^{\star}(\varphi^z)$$

$$\text{avec} : \left\{ \begin{array}{rcl} R^{\star}(\varphi^x) & = & R(\varphi^x) + \rho(\varphi^x) \\ R^{\star}(\varphi^y) & = & R(\varphi^y) + \rho(\varphi^y) \\ R^{\star}(\varphi^z) & = & R(\varphi^z) + \rho(\varphi^z) \end{array} \right.$$

Compte tenu de l'indépendance des composantes $\varphi^x$, $\varphi^y$ et $\varphi^z$, on en conclut que :

$$R^{\star}(\vec{\varphi}) \text{ minimum } \Longleftrightarrow \left\{ \begin{array}{l} R^{\star}(\varphi^x) \text{ minimum} \\ R^{\star}(\varphi^y) \text{ minimum} \\ R^{\star}(\varphi^z) \text{ minimum} \end{array} \right.$$

La minimisation du critère vectoriel DSI est donc équivalente à la minimisation des trois critères DSI correspondant aux trois composantes du vecteur courant $\vec{\varphi}$ .

### 2.7.4 Remarque

Si la surface $S$ est composée de plusieurs morceaux disjoints, alors le théorème présenté au paragraphe 2.3 nous garantit que notre problème admet une solution unique dés lors qu'il y a au moins un sommet de facette ayant une position connue sur chaque morceau.

### 2.7.5 Utilisation interactive

En modélisation géométrique, l'objectif est de définir interactivement la position dans l'espace du graphe $G$ associé à l'ensemble des noeuds $\Omega$. Pour ce faire, on suppose connue une forme initiale et l'utilisateur déplace quelques noeuds et modifie interactivement un certain nombre de contraintes (noeuds de contrôles et données imprécises); ces modifications sont alors propagées aux noeuds $i \in I$ en utilisant la forme vectorielle de la méthode DSI.

Nous avons montré dans les paragraphes précédents que ce problème se décomposait en trois sous-problèmes correspondant aux trois coordonnées $(x, y, z)$ et la meilleure façon de résoudre ces trois problèmes est d'utiliser l'algorithme itératif associé à la forme locale des équations DSI correspondantes.

D'une étape à l'autre du processus de modélisation on peut modifier non seulement la position des coordonnées des noeuds de contrôle mais l'ensemble $L$ lui même peut être changé si nécessaire; par exemple, si nous voulons appliquer DSI seulement à un sous ensemble $\Gamma$ de noeuds, alors nous devons choisir $L$ de telle façon que $L \supset \overline{\Gamma}$ où $\overline{\Gamma}$ désigne l'ensemble complémentaire de $\Gamma$ dans $\Omega$.

De plus, compte tenu de la décomposition de notre problème en trois sous-problèmes résolus indépendamment et correspondant aux trois coordonnées, on peut, si nécessaire, définir des sous ensembles différents $L_x$, $L_y$ et $L_z$ pour chacune de ces coordonnées; par exemple, si les coordonnées $(x, y)$ ne doivent pas être modifiées, alors il suffit de prendre $L_x$ et $L_y$ égaux à l'ensemble $\Omega$ tout entier.

### 2.7.6 Prise en compte de données géométriques imprécises

Initialement, la méthode DSI à été conçue pour modéliser les surfaces complexes rencontrées dans le domaine des sciences naturelles comme par exemple la géologie et la biologie. Dans ce cas, on doit généralement prendre en compte de nombreuses données imprésices comme par exemple :

$$\left\{ \begin{array}{l} \bullet \text{ des noeuds de contrôle flous} \\ \bullet \text{ des données vectorielles floues} \end{array} \right.$$

**Noeuds de contrôle flous**

Par définition, nous appellerons "noeud de contrôle flou" tout noeud $\lambda \in I$ dont la position $\vec{\varphi}_\lambda$ est connue comme étant approximativement égale à un vecteur donné $\tilde{\vec{\varphi}}_\lambda$ et ceci avec un certain degré de certitude $\overline{\omega}_\lambda^2$. En se référant au paragraphe 2.5.1, on voit que l'on peut prendre en compte une telle information imprécise à l'aide de la méthode DSI en introduisant les contraintes suivantes dans les critères $R^*(\varphi^x)$, $R^*(\varphi^y)$ et $R^*(\varphi^z)$ :

$$\begin{cases} \varphi_\lambda^x \simeq \tilde{\varphi}_\lambda^x \\ \varphi_\lambda^y \simeq \tilde{\varphi}_\lambda^y \\ \varphi_\lambda^z \simeq \tilde{\varphi}_\lambda^z \end{cases}$$

**Contraintes vectorielles floues**

Dans de nombreuses applications, il est nécessaire de contrôler le vecteur ($\vec{\varphi}_\mu$ - $\vec{\varphi}_\lambda$) joignant deux noeuds $\vec{\varphi}_\lambda$ et $\vec{\varphi}_\mu$ d'une surface. Par définition, une telle contrainte sera appelée "contrainte vectorielle floue" si elle doit être satisfaite avec un certain degré de certitude $\overline{\omega}_{\lambda\mu}^2$. Quelques exemples de contraintes de ce type sont presentés sur la Figure 2.

En pratique, au moins un des deux points $\vec{\varphi}_\lambda$ où $\vec{\varphi}_\mu$, a une position inconnue, par exemple celui correspondant à $\lambda \in I$, et parmi les cas importants, on peut citer les deux suivants :
- Le premier cas correspond aux Figures 2.a et 2.b où l'on désire contrôler la forme d'un objet de telle façon que ($\vec{\varphi}_\mu$ - $\vec{\varphi}_\lambda$) soit égal à un vecteur donné $\vec{\Delta}_{\lambda\mu}$.
- Le second cas correspond à la Figure 2.c où l'on désire contrôler la forme d'un objet de telle façon que chaque ($\vec{\varphi}_\mu$ - $\vec{\varphi}_\lambda$) soit orthogonal à un vecteur donné $\vec{V}$. Cette situation se présente lorsque l'on veut contrôler le vecteur normal à une surface; dans ce cas, si $\vec{V}$ est le vecteur normal au noeud $\lambda$ de la surface, il est judicieux de contrôler son orthogonalité avec chacun de ces vecteurs ($\vec{\varphi}_\mu$ - $\vec{\varphi}_\lambda$) pour tout $\mu \in N(\lambda)$.

Si l'on se reporte au paragraphe 2.5.2, le premier cas est facilement pris en compte par la méthode DSI en introduisant les contraintes suivantes dans les critères $R^*(\varphi^x)$, $R^*(\varphi^y)$ et $R^*(\varphi^z)$ avec un coefficient de pondération égal à $\overline{\omega}_{\lambda\mu}^2$ :

$$\begin{cases} (\varphi_\mu^x - \varphi_\lambda^x) \simeq \Delta_{\lambda\mu}^x \\ (\varphi_\mu^y - \varphi_\lambda^y) \simeq \Delta_{\lambda\mu}^y \\ (\varphi_\mu^z - \varphi_\lambda^z) \simeq \Delta_{\lambda\mu}^z \end{cases}$$

Pour le second cas, le problème n'est pas trés différent car on a :

$$(\vec{\varphi}_\mu - \vec{\varphi}_\lambda) \cdot \vec{V} \simeq 0$$

$$\Longleftrightarrow \begin{cases} (\varphi_\mu^x - \varphi_\lambda^x) \simeq -\{V^y \cdot (\varphi_\mu^y - \varphi_\lambda^y) + V^z \cdot (\varphi_\mu^z - \varphi_\lambda^z)\}/V^x \\ (\varphi_\mu^y - \varphi_\lambda^y) \simeq -\{V^z \cdot (\varphi_\mu^z - \varphi_\lambda^z) + V^x \cdot (\varphi_\mu^x - \varphi_\lambda^x)\}/V^y \\ (\varphi_\mu^z - \varphi_\lambda^z) \simeq -\{V^x \cdot (\varphi_\mu^x - \varphi_\lambda^x) + V^y \cdot (\varphi_\mu^y - \varphi_\lambda^y)\}/V^z \end{cases}$$

Dans ce cas, nous suggérons de prendre les valeurs des termes $(\varphi_\lambda^x, \varphi_\lambda^y, \varphi_\lambda^z)$ et $(\varphi_\mu^x, \varphi_\mu^y, \varphi_\mu^z)$ intervenant dans les membres de droite de ces équations égales aux valeurs correspondantes obtenues à l'itération précédente; pour se ramener aux cas précédent, il suffit alors de poser :

$$\Delta_{\lambda\mu}^x = - \{V^y \cdot (\varphi_\mu^y - \varphi_\lambda^y) + V^z \cdot (\varphi_\mu^z - \varphi_\lambda^z)\}/V^x$$
$$\Delta_{\lambda\mu}^y = - \{V^z \cdot (\varphi_\mu^z - \varphi_\lambda^z) + V^x \cdot (\varphi_\mu^x - \varphi_\lambda^x)\}/V^y$$
$$\Delta_{\lambda\mu}^z = - \{V^z \cdot (\varphi_\mu^x - \varphi_\lambda^x) + V^y \cdot (\varphi_\mu^y - \varphi_\lambda^y)\}/V^z$$

**Remarque**

Dans la prise en compte des données imprécises présentée ci-dessus, nous n'avons utilisé qu'un seul coefficient de certitude $\overline{\omega}_{\lambda\mu}^2$ commun aux trois composantes de ces contraintes. En pratique, ceci n'est pas une obligation et, compte tenu de l'indépendance des trois composantes, il est toujours possible d'utiliser trois coefficients de certitude différents $\overline{\omega}_{\lambda\mu}^{x2}, \overline{\omega}_{\lambda\mu}^{y2}$ et $\overline{\omega}_{\lambda\mu}^{z2}$ correspondant à ces trois composantes.

**2.8 Généralisations**

Avant de conclure, mentionnons quelques généralisations évidentes :
- La methode DSI peut être utilisée avec n'importe quels types de facettes polygonales. En pratique, nous conseillons d'utiliser des triangles car ce sont les polygones les plus simples et les plus faciles à manipuler dans un ordinateur.
- La méthode DSI peut être utilisée pour estimer des courbes polygonales dans un espace 3D; dans ce cas, les triangles sont remplacés par des segments.

**2.9 CONCLUSION**

La forme locale de l'équation DSI constitue un outil simple et puissant pour modéliser les surfaces complexes que l'on peut rencontrer par exemple en géologie où en biologie. En particulier :
- Le maillage utilisé pour modéliser une surface peut être complètement libre et il est possible d'utiliser des algorithmes automatiques pour les construire; par exemple, si le maillage est composé de triangles, on peut utiliser des algorithmes dérivés de la méthode de Delaunay (voir ref. [2] et [5]). De plus, la taille du maillage peut facilement être ajustée localement en fonction de la complexité de la surface.
- La méthode DSI permet de prendre en compte un trés grand nombre de données précises ou imprécises. Cette possibilité est particulièrement intéressante dans le domaine des sciences naturelles où l'objectif n'est pas de générer des surfaces esthétiques mais d'ajuster ces surfaces aux données précises et imprécises de façon cohérente et efficace.
- L'algorithme dérivé de l'équation DSI locale est suffisamment rapide pour permettre une utilisation interactive.

Nous n'avons rien dit sur la représentation des facettes elles-mêmes. Selon les applications, on peut prendre des facettes planes mais, si cela est nécessaire, il est toujours possible de représenter ces facettes par des morceaux de surfaces non planes interpolant les noeuds du maillage (voir ref. [4]).

**Revendications**

1. Procédé d'obtention d'une modélisation d'une surface (S) représentative notamment de l'interface entre deux zones de natures ou de propriétés différentes dans un corps à trois dimensions tel qu'une formation géologique ou un corps vivant, du type comprenant les étapes consistant à :
   - obtenir au moyen d'appareils de mesure un ensemble de données géométriques relatives à la surface et associées respectivement à des points donnés de ladite surface;
   - effectuer un maillage de la surface, constitué de facettes polygonales ayant pour sommets les noeuds du maillage, de telle sorte que l'ensemble desdits points donnés soit un sous-ensemble des noeuds ( $\vec{\varphi}_k$ ) du maillage;
   - mémoriser les informations suivantes :
     * les coordonnées $(\varphi_k^x, \varphi_k^y, \varphi_k^z)$ de chaque noeud,
     * le nombre (nb-sat) de noeuds satellites du noeud considéré, chacun de ces noeuds satellites

( $\vec{\varphi_j}$ ) étant lié avec le noeud considéré ( $\vec{\varphi_k}$ ) par un côté d'une desdites facettes polygonales,

* le cas échéant des données géométriques (const) associées audit noeud considéré,

- pour chaque noeud du maillage, définir un indice de rugosité local (R($\varphi$|k)) obtenu à partir d'une somme pondérée des coordonnées actuelles du noeud et de ses satellites;

- ajuster de façon itérative les coordonnées de chacun des noeuds dont on ne connaît pas les coordonnées précises en utilisant une combinaison des indices de rugosité locaux des différents noeuds, et

- créer une représentation de la surface à partir des coordonnées ajustées ($\varphi_k{}^x$, $\varphi_k{}^y$, $\varphi_k{}^z$) de chacun des noeuds;

**caractérisé en ce que :**

- lesdites informations sont mémorisées à des adresses de la mémoire spécifiques à chaque noeud ( $\vec{\varphi_k}$ ) du maillage;

- lesdites informations incluent également une information (sat) permettant d'accéder aux adresses spécifiques desdits noeuds satellites et par suite aux informations relatives à ces derniers, et

- ladite étape d'ajustement de coordonnées comprend les sous-étapes consistant à :

  · définir la somme (R*($\varphi$)) d'un indice de rugosité global (R($\varphi$)), constitué par l'addition des indices de rugosité locaux associés à chaque noeud, et d'un indice global ($\rho(\varphi)$) de violation desdites données géométriques;

  · utiliser pour chaque noeud dont on ajuste les coordonnées l'addition d'une combinaison des coordonnées actuelles des satellites et des satellites des satellites dudit noeud et d'une combinaison des données géométriques associées audit noeud, et amener ladite somme (R*($\varphi$)) de l'indice de rugosité global et de l'indice global de violation desdites données géométriques à un minimum.

2.  Procédé selon la revendication 1, caractérisé en ce qu'on effectue un maillage triangulaire de la surface.

3.  Procédé selon l'une des revendications 1 et 2, caractérisé en ce que l'étape d'ajustement comprend trois sous-étapes distinctes mettant en jeu respectivement les trois coordonnées spatiales ($\varphi_k{}^x$, $\varphi_k{}^y$, $\varphi_k{}^z$) des noeuds et des satellites.

4.  Procédé selon l'une des revendications 1 à 3, caractérisé en ce que les données géométriques comprennent des données de coordonnées ( $\vec{\varphi_k}$ ) des noeuds.

5.  Procédé selon l'une des revendications 1 à 4, caractérisé en ce que les données géométriques comprennent des données de vecteurs ( $\vec{\varphi_\lambda}$ - $\vec{\varphi_k}$ ) entre deux noeuds donnés.

6.  Procédé selon l'une des revendications 1 à 5, caractérisé en ce que les données géométriques comprennent des données de vecteur normal ( $\vec{V}$ ) à la surface à modéliser.

7.  Procédé selon l'une des revendications 4 à 6, caractérisé en ce qu'au moins certaines desdites données géométriques sont assorties d'un coefficient ($\overline{\omega}_\lambda$ $\overline{\omega}_{\lambda\mu}$) représentatif du degré de certitude avec lequel ces données sont connues et en ce que l'indice de violation des données intègre lesdits coefficients de certitude.

8.  Procédé selon l'une des revendications précédentes, caractérisé en ce qu'il comprend en outre les étapes consistant à mesurer des propriétés du corps à trois dimensions dans la région d'au moins certains noeuds du maillage et à mémoriser les mesures obtenues (info) aux adresses spécifiques aux noeuds considérés.

9.  Procédé selon l'une des revendications précédentes, caractérisé en ce que, dans chaque coefficient de rugosité local, le rapport entre le poids ($v^\alpha(k)$; $\alpha=k$) du noeud concerné et le poids ($v^\alpha(k)$; $\alpha \in \Lambda(k)$) d'un satellite quelconque dudit noeud est égal au nombre (nk) de satellites dudit noeud, affecté d'un signe négatif.

10.  Procédé selon la revendication 9, caractérisé en ce que ladite combinaison pondérée utilise les mêmes poids.

11. Procédé selon l'une quelconque des revendications précédentes, caractérisé en ce que l'addition des indices de rugosité locaux est une somme pondérée.

12. Procédé selon la revendication 11, rattachée à la revendication 10, caractérisé en ce que ladite combinaison pondérée utilise également des poids ($\mu(k)$) associés aux coefficients de rugosité locaux.

13. Procédé selon l'une des revendications précédentes, caractérisé en ce que la surface est une surface de séparation entre deux milieux de propriétés ou de natures différentes dans une formation géologique et en ce qu'on utilise la représentation de la surface pour optimiser l'exploration de richesses souterraines de ladite formation.

14. Procédé selon l'une des revendications 1 à 13, caractérisé en ce que la représentation de la surface est une représentation graphique sur un support plan.

15. Procédé selon l'une des revendications 1 à 13, caractérisé en ce que la représentation de la surface est une maquette en trois dimensions.

16. Dispositif pour l'obtention d'une modélisation d'une surface (S) représentative notamment de l'interface entre deux zones de natures ou de propriétés différentes dans un corps à trois dimensions tel qu'une formation géologique ou un corps vivant, du type comprenant :
   - des moyens de mesure permettant d'obtenir un ensemble de données géométriques relatives à la surface et associées respectivement à des points donnés de ladite surface;
   - des moyens pour effectuer un maillage de la surface, constitué de facettes polygonales ayant pour sommets les noeuds du maillage, de telle sorte que l'ensemble desdits points donnés soit un sous-ensemble des noeuds ( $\vec{\varphi}_k$ ) du maillage;
   - une mémoire contenant les informations suivantes :
      * les coordonnées ($\varphi_k{}^x$, $\varphi_k{}^y$, $\varphi_k{}^z$) du noeud considéré,
      * le nombre (nb-sat) de noeuds satellites du noeud considéré, chacun de ces noeuds satellites ( $\vec{\varphi}_j$ ) étant lié avec le noeud considéré ( $\vec{\varphi}_k$ ) par un côté d'une desdites facettes polygonales,
      * le cas échéant des données géométriques (const) associées audit noeud considéré,
   - des moyens de calcul destinés, pour chaque noeud du maillage, à définir un indice de rugosité local ($R(\varphi \mid k)$) obtenu à partir d'une somme pondérée des coordonnées actuelles du noeud et de ses satellites, et à ajuster de façon itérative les coordonnées de chacun des noeuds dont on ne connaît pas les coordonnées précises en utilisant une combinaison des indices de rugosité locaux des différents noeuds, et
   - des moyens pour créer une représentation de la surface à partir des coordonnées ajustées ($\varphi_k{}^x$, $\varphi_k{}^y$, $\varphi_k{}^z$) de chacun des noeuds;
      **caractérisé en ce que :**
   - lesdites informations sont mémorisées à des adresses de la mémoire spécifiques à chaque noeud ( $\vec{\varphi}_k$ ) du maillage;
   - lesdites informations incluent également une information (sat) permettant d'accéder aux adresses spécifiques desdits noeuds satellites et par suite aux informations relatives à ces derniers, et
   - les moyens de calcul comprennent également :
      · des moyens pour définir la somme ($R^*(\varphi)$) d'un indice de rugosité global ($R(\varphi)$), constitué par l'addition des indices de rugosité locaux ($R(\varphi \mid k)$) respectivement associés à chacun des noeuds et d'un indice global ($\rho(\varphi)$) de violation desdites données géométriques;
      · des moyens pour amener à un minimum ladite somme ($R^*(\varphi)$) de l'indice de rugosité global et de l'indice global de violation desdites données géométriques.

**Patentansprüche**

1. Verfahren zum Erhalten einer Modellierung einer Oberfläche (S), welche eine Grenzfläche zwischen zwei natürlichen Bereichen oder Bereichen mit unterschiedlichen Eigenschaften in einem dreidimensionalen Körper, wie einer geologischen Formation oder einem lebenden Körper, darstellt, mit den folgenden Verfahrensschritten:

- Erzeugen einer Anordnung von geometrischen Daten relativ zu der Oberfläche, welche jeweils gegebenen Punkten dieser Oberfläche zugeordnet sind, mit Hilfe eines Meßgerätes,
- Bilden eines Netzwerkes der Oberfläche, welches aus polygonalen Facetten aufgebaut ist, deren Scheitel durch die Knoten des Netzwerkes gebildet werden, so daß die Anordnung aus den gegebenen Punkten eine Unteranordnung der Knoten ( $\vec{\varphi}_k$ ) des Netzwerkes ist,
- Speichern der folgenden Informationen:
    * die Koordinaten ($\varphi_k x$, $\varphi_k y$, $\varphi_k z$) jedes Knotens,
    * die Anzahl (nb-sat) der Satellitenknoten des betrachteten Knotens, wobei jeder dieser Satellitenknoten ( $\vec{\varphi}_j$ ) mit dem betrachteten Knoten ( $\vec{\varphi}_k$ ) über eine Seite einer der polygonalen Facetten verbunden ist,
    * gegebenenfalls die geometrischen Daten (const), welche diesem betrachteten Knoten zugeordnet sind,
- für jeden Knoten des Netzwerkes, Definieren eines lokalen Indexes der Rauhigkeit (R($\varphi$|k)), welcher ausgehend von einer gewichteten Summe der momentanen Koordinaten des Knotens und seiner Satelliten erhalten wird,
- Einstellen der Koordinaten jedes Knotens, von dem man die genauen Koordinaten nicht kennt, auf iterative Weise, indem man eine Kombination der lokalen Indizes der Rauhigkeit der unterschiedlichen Knoten verwendet, und
- Erzeugen einer Darstellung der Oberfläche ausgehend von den eingestellten Koordinaten ($\varphi_k x$, $\varphi_k y$, $\varphi_k z$) jedes Knotens,

dadurch **gekennzeichnet**, daß

- die Informationen bei Adressen des Speichers gespeichert werden, welche für jeden Knoten ( $\vec{\varphi}_k$ ) des Netzwerkes spezifisch sind,
- die Informationen auch eine Information (sat) umfassen, welche den Zugriff auf die spezifischen Adressen der Satellitenknoten und nachfolgend auf die auf letztere bezogenen Informationen erlauben, und
- der Schritt, mit dem die Koordinaten eingestellt werden, die folgenden Unterschritte umfaßt:
    * Definieren der Summe (R*($\varphi$)) eines globalen Indexes der Rauhigkeit (R($\varphi$)), welche durch die Addition der jedem Knoten zugeordneten lokalen Indizes der Rauhigkeit und eines globalen Indexes ($\rho(\varphi)$) der Verletzung dieser geometrischen Daten gebildet wird,
    * für jeden Knoten, für den die Koordinaten eingestellt werden, Verwenden der Addition einer Kombination der aktuellen Koordinaten der Satelliten und der Satelliten der Satelliten dieses Knotens und einer Kombination der jedem Knoten zugeordneten geometrischen Daten und Einstellen der Summe (R*($\varphi$)) des globalen Indexes der Rauhigkeit und des globalen Indexes der Verletzung dieser geometrischen Daten auf ein Minimum.

2. Verfahren nach Anspruch 1, dadurch **gekennzeichnet**, daß ein trigonales Netzwerk der Oberfläche gebildet wird.

3. Verfahren nach einem der Ansprüche 1 und 2, dadurch **gekennzeichnet**, daß der Einstellschritt drei verschiedene Unterschritte umfaßt, welche jeweils die drei räumlichen Koordinaten ($\varphi_k x$, $\varphi_k y$, $\varphi_k z$) der Knoten und der Satelliten ins Spiel bringen.

4. Verfahren nach einem der Ansprüche 1 bis 3, dadurch **gekennzeichnet**, daß die geometrischen Daten Daten der Koordinaten ( $\vec{\varphi}_k$ ) der Knoten umfassen.

5. Verfahren nach einem der Ansprüche 1 bis 4, dadurch **gekennzeichnet**, daß die geometrischen Daten Daten der Vektoren ( $\vec{\varphi}_\lambda$ - $\vec{\varphi}_k$ ) zwischen zwei gegebenen Knoten umfassen.

6. Verfahren nach einem der Ansprüche 1 bis 5, dadurch **gekennzeichnet**, daß die geometrischen Daten Daten des auf die zu modellierende Oberfläche senkrechten Vektors ($\vec{V}$) umfassen.

7. Verfahren nach einem der Ansprüche 4 bis 6, dadurch **gekennzeichnet**, daß mindestens einige bestimmte geometrische Daten einem Koeffizienten ($\bar{\omega}_\lambda$ $\bar{\omega}_\lambda \mu$) zugeordnet sind, der einem Wahrscheinlichkeitsgrad entspricht, mit dem diese Daten bekannt sind, und daß der Index der Verletzung der Daten diese Wahrscheinlichkeits-Koeffizienten integriert.

8. Verfahren nach einem der vorangehenden Ansprüche, dadurch **gekennzeichnet**, daß es ferner die folgenden Schritte umfaßt: Messen der Eigenschaften des dreidimensionalen Körpers in dem Bereich mit mindestens einigen bestimmten Knoten des Netzwerkes und Speichern der erhaltenen Meßwerte (info) bei den für die betrachteten Knoten spezifischen Adressen.

9. Verfahren nach einem der vorangehenden Ansprüche, dadurch **gekennzeichnet**, daß bei jedem lokalen Koeffizienten der Rauhigkeit das Verhältnis zwischen dem Gewicht ($v^\alpha(k)$; $\alpha=k$) des betrachteten Knotens und dem Gewicht ($v^\alpha(k)$; $\alpha\in\Lambda(k)$) irgendeines Satelliten dieses Knotens gleich der Anzahl (nk) der Satelliten dieses Knotens, mit einem negativen Vorzeichen behaftet, ist.

10. Verfahren nach Anspruch 9, dadurch **gekennzeichnet**, daß die gewichtete Verknüpfung dieselben Gewichte verwendet.

11. Verfahren nach irgendeinem der vorangehenden Ansprüche, dadurch **gekennzeichnet**, daß die Addition der lokalen Indizes der Rauhigkeit eine gewichtete Summe ist.

12. Verfahren nach Anspruch 11 und Anspruch 10, dadurch **gekennzeichnet**, daß die gewichtete Verknüpfung auch Gewichte ($\mu(k)$) verwendet, welche den lokalen Koeffizienten der Rauhigkeit zugeordnet sind.

13. Verfahren nach einem der vorangehenden Ansprüche, dadurch **gekennzeichnet**, daß die Oberfläche eine Trennfläche zwischen zwei Gebieten mit unterschiedlichen Eigenschaften oder Eigenarten in einer geologischen Formation ist, und daß man die Darstellung der Oberfläche zum Optimieren der Erforschung der Bodenschätze dieser Formation verwendet.

14. Verfahren nach einem der Ansprüche 1 bis 13, dadurch **gekennzeichnet**, daß die Darstellung der Oberfläche eine graphische Darstellung auf einer Trägerfläche ist.

15. Verfahren nach einem der Ansprüche 1 bis 13, dadurch **gekennzeichnet**, daß die Darstellung der Oberfläche ein dreidimensionales Muster ist.

16. Vorrichtung zum Erhalten der Modellierung einer Oberfläche (S), welche eine Grenzfläche zwischen zwei natürlichen Bereichen oder Bereichen mit unterschiedlichen Eigenschaften in einem dreidimensionalen Körper, wie einer geologischen Formation oder einem lebenden Körper, darstellt, mit:
   - einer Meßeinrichtung zum Erzeugen einer Anordnung von geometrischen Daten relativ zu der Oberfläche, welche jeweils gegebenen Punkten dieser Oberfläche zugeordnet sind,
   - Mitteln zum Bilden eines Netzwerkes der Oberfläche, welches aus polygonalen Facetten aufgebaut ist, deren Scheitel durch die Knoten des Netzwerkes gebildet werden, so daß die Anordnung aus den gegebenen Punkten eine Unteranordnung der Knoten ($\vec{\varphi}_k$) des Netzwerkes ist,
   - einem Speicher, welcher die folgenden Informationen enthält:
      * die Koordinaten ($\varphi_k x$, $\varphi_k y$, $\varphi_k z$) jedes Knotens,
      * die Anzahl (nb-sat) der Satellitenknoten des betrachteten Knotens, wobei jeder dieser Satellitenknoten ($\vec{\varphi}_j$) mit dem betrachteten Knoten ($\vec{\varphi}_k$) über eine Seite einer der polygonalen Facetten verbunden ist,
      * gegebenenfalls die geometrischen Daten (const), welche diesen betrachteten Knoten zugeordnet sind,
   - einer Recheneinrichtung, um für jeden Knoten des Netzwerkes einen lokalen Index der Rauhigkeit ($R(\varphi\,|\,k)$) zu definieren, welcher ausgehend von einer gewichteten Summe der momentanen Koordinaten des Knotens und seiner Satelliten erhalten wird, und um die Koordinaten jedes Knotens, von dem man die genauen Koordinaten nicht kennt, auf iterative Weise einzustellen, indem man eine Kombination der lokalen Indizes der Rauhigkeit der unterschiedlichen Knoten verwendet, und

- Mitteln zum Erzeugen einer Darstellung der Oberfläche ausgehend von den eingestellten Koordinaten ($\varphi_k x$, $\varphi_k y$, $\varphi_k z$) jedes Knotens,

dadurch **gekennzeichnet**, daß

- die Informationen bei Adressen des Speichers gespeichert werden, welche für jeden Knoten

( $\vec{\varphi}_k$ ) des Netzwerkes spezifisch sind,

- die Informationen auch eine Information (sat) umfassen, welche den Zugriff auf die spezifischen Adressen der Satellitenknoten und nachfolgend auf die auf letztere bezogenen Informationen erlauben, und
- die Recheneinrichtung ferner folgende Merkmale aufweist:
  * Mittel zum Definieren der Summe (R*($\varphi$)) eines globalen Indexes der Rauhigkeit (R($\varphi$)), welche durch die Addition der jedem Knoten zugeordneten lokalen Indizes der Rauhigkeit und eines globalen Indexes ($\rho(\varphi)$) der Verletzung dieser geometrischen Daten gebildet wird,
  * Mittel zum Einstellen der Summe (R*($\varphi$)) des globalen Indexes der Rauhigkeit und des globalen Indexes der Verletzung dieser geometrischen Daten auf ein Minimum.

## Claims

1. Process for modelling a surface (S) representing for example the interface between two areas of different kinds or with different properties in a three-dimensional body such as a geological formation or a living body, of the type comprising the steps of:
   - obtaining by means of measuring apparatus a set of geometrical data relating to the surface and associated with respective points on said surface;
   - meshing the surface with polygonal facets the vertices of which are nodes of the mesh, so that all said points are a subset of the nodes ( $\vec{\varphi}_k$ ) of the mesh;
   - storing the following data:
     * the coordinates ($\varphi_k{}^x$, $\varphi_k{}^y$, $\varphi_k{}^z$) of each node,
     * the number (nb-sat) of satellite nodes of the node in question, each of the satellite nodes

     ( $\vec{\varphi}_j$ ) being linked to the node in question ( $\vec{\varphi}_k$ ) by one side of one of said polygonal facets,
     * if necessary, geometrical data (const) associated with said node in question,
   - for each node of the mesh, defining a local roughness index (R($\varphi$|k)) derived from a weighted sum of the actual coordinates of the node and of its satellites,
   - fitting the coordinates of each node for which the precise coordinates are not known by an iterative method using a combination of the local roughness indices of the various nodes, and
   - creating a representation of the surface from the fitted coordinates ($\varphi_k{}^x$, $\varphi_k{}^y$, $\varphi_k{}^z$) of each node; characterised in that:

   - said data is stored at specific memory addresses for each node ( $\vec{\varphi}_k$ ) of the mesh;
   - said data further includes data (sat) providing access to the specific addresses of said satellite nodes and consequently to the data relating thereto, and
   - said coordinate fitting step includes the following sub-steps:
     . defining the sum (R*($\varphi$)) of a global roughness index (R($\varphi$)) obtained by summing the local roughness indices associated with each node and a global index ($\rho(\varphi)$) of violation of said geometrical data,
     . using for each node for which the coordinates are fitted the addition of a combination of the actual coordinates of the satellites and of the satellites of the satellites of said node and a combination of the geometrical data associated with said node, and minimising said sum (R*($\varphi$)) of the global roughness index and the global index of violation of said geometrical data.

2. Process according to claim 1, characterised in that the surface is meshed using triangles.

3. Process according to claim 1 or claim 2, characterised in that the fitting step comprises three separate sub-steps respectively using the three spatial coordinates ($\varphi_k{}^x$, $\varphi_k{}^y$, $\varphi_k{}^z$) of the nodes and of the satellites.

4. Process according to any one of claims 1 to 3, characterised in that the geometrical data includes node coordinate data ( $\vec{\varphi}_k$ ).

5. Process according to any one of claims 1 to 4, characterised in that the geometrical data includes data of vectors ( $\vec{\varphi}_\lambda$ - $\vec{\varphi}_k$ ) between two given nodes.

6. Process according to any one of claims 1 to 5, characterised in that the geometrical data includes data of the vector ( $\vec{V}$ ) normal to the surface to be modelled.

7. Process according to any one of claims 4 to 6, characterised in that at least some of said geometrical data is associated with a coefficient ($\overline{\omega}_\lambda$ $\overline{\omega}_\lambda\mu$) representing the certainty factor with which said data is known and in that the data violation index incorporates said certainty factors.

8. Process according to any one of the preceding claims, characterised in that it further comprises the steps of measuring properties of the three-dimensional body in the region of at least some nodes of the mesh and storing the measurements obtained (info) at the addresses specific to the nodes in question.

9. Process according to any one of the preceding claims, characterised in that, in each local roughness coefficient, the ratio between the weight ($v^\alpha(k)$; $\alpha = k$) of the node concerned and the weight ($v^\alpha(k)$; $\alpha \in \Lambda(k)$) of any satellite of said node is equal to the number (nk) of satellites of said node, with a negative sign.

10. Process according to claim 9, characterised in that said weighted combination uses the same weights.

11. Process according to any one of the preceding claims, characterised in that the sum of the local roughness indices is a weighted sum.

12. Process according to claim 11 in combination with claim 10, characterised in that said weighted combination uses also weights ($\mu(k)$) associated with the local roughness coefficients.

13. Process according to any one of the preceding claims, characterised in that the surface is a surface separating two media with different properties or of different kinds in a geological formation and in that the representation of the surface is used to optimise prospecting for underground resources of said formation.

14. Process according to any one of claims 1 to 13, characterised in that the representation of the surface is a graphical representation on a flat medium.

15. Method according to any one of claims 1 to 13, characterised in that the representation of the surface is a three-dimensional model.

16. Device for modelling a surface (S) representing for example the interface between two areas of different kinds or with different properties in a three-dimensional body such as a geological formation or a living body, of the type comprising:
   - measuring means for obtaining a set of geometrical data relating to the surface and associated with respective points on said surface;
   - means for meshing the surface with polygonal facets the vertices of which are nodes of the mesh, so that all said points are a subset of the nodes ( $\vec{\varphi}_k$ ) of the mesh;
   - a memory containing the following data:
     * the coordinates ($\varphi_k{}^x$, $\varphi_k{}^y$, $\varphi_k{}^z$) of the node in question,
     * the number (nb-sat) of satellite nodes of the node in question, each of the satellite nodes ( $\vec{\varphi}_j$ ) being linked to the node in question ( $\vec{\varphi}_k$ ) by one side of one of said polygonal facets,
     * if necessary, geometric data (const) associated with said node in question,
   - calculation means for defining, for each node of the mesh, a local roughness index ($R(\varphi \,|\, k)$ obtained from a weighted sum of the actual coordinates of the node and of its satellites, and for fitting the coordinates of each node for which the precise coordinates are not known by an iterative method using a combination of the local roughness indices of the various nodes, and

- means for creating a representation of the surface from the fitted coordinates ($\varphi_k{}^x$, $\varphi_k{}^y$, $\varphi_k{}^z$) of each node,

           characterised in that:

- said data is stored at specific memory addresses for each node ( $\vec{\varphi}_k$ ) of the mesh;
- said data further includes data (sat) providing access to the specific addresses of said satellite nodes and consequently to the data relating thereto, and
- said calculation means further include:
  - . means for defining the sum ($R^*(\varphi)$) of a global roughness index ($R(\varphi)$) obtained by summing respective local roughness indices ($R(\varphi | k)$) associated with each node and a global index ($\rho(\varphi)$) of violation of said geometrical data;
  - . means for minimising said sum ($R^*(\varphi)$) of the global roughness index and the global index of violation of said geometrical data.

Figure 1

noeud de contrôle
noeud libre

Figure 2(a)

Figure 2(b)

Figure 2(c)

Figure 2(d)

Figure 3

Figure 4

Atome A(k)

Figure 5

46

sat
(= i)

adresse (i) — $A^*(j_1)$ → $A(j_1)$

adresse (i+1) — $A^*(j_2)$ → $A(j_2)$

tableau
de
$n_k$ cases

adresse $(i+n_k)$ — $A^*(j_{nk})$ → $A(j_{nk})$

**Figure 6**

sat
(= $i_1$)

adresse ($i_1$) | next (= adresse $i_2$) | $A^*(j_1)$ → $A(j_1)$

adresse ($i_2$) | next (= adresse $i_3$) | $A^*(j_2)$ → $A(j_2)$

adresse ($i_{nk}$) | next (= code fin) | $A^*(j_{nk})$ → $A(j_{nk})$

**Figure 7**

| next | contrainte |
|------|-----------|

**Figure 8**

**Atome A(k)**

| noyau | nb-sat | sat | const ($=$ adresse $c_1$) | info |
|-------|--------|-----|---------------------------|------|

**Liste des contraintes**

adresse ($c_1$)

| next ($=$ adresse $c_2$) | contrainte |
|--------------------------|-----------|

adresse ($c_2$)

| next ($=$ adresse $c_3$) | contrainte |
|--------------------------|-----------|

| next ($=$ code fin) | contrainte |
|---------------------|-----------|

**Figure 9**

A*(1)
(= adresse a) → A(1)

A*(2)
(= adresse a+1) → A(2)

...

A*(N)
(= adresse a+N-1) → A(N)

**Figure 10**

A*(1)
(= adresse $a_1$) → next (= adresse $a_2$) | Atom A(1)

A*(2)
(= adresse $a_2$) → next (= adresse $a_3$) | Atom A(2)

...

A*(N)
(= adresse $a_N$) → next (= code fin) | Atom A(N)

**Figure 11**